# EUROPÄISCHE PATENTSCHRIFT

(11) **EP 1 317 570 B1**
(45) Veröffentlichungstag und Bekanntmachung des Hinweises auf die Patenterteilung: **06.04.2005**
(21) Anmeldenummer: 01984658.3
(22) Anmeldetag: 15.09.2001
(51) Int. Cl.: C12Q 1/68

(54) **PCR-REAKTIONSGEMISCH FÜR FLUORESZENZ-BASIERENDE GENEXPRESSIONS- UND GENMUTATIONSANALYSEN**
PCR REACTION MIXTURE FOR FLUORESCENCE-BASED GENE ANALYSIS AND GENE MUTATION ANALYSIS
MELANGE REACTIONNEL DE PCR POUR ANALYSES DE L'EXPRESSION GENIQUE ET DE LA MUTATION GENIQUE FONDEES SUR LA FLUORESCENCE

(30) Priorität: 15.09.2000 DE 10046079
(43) Veröffentlichungstag der Anmeldung: 11.06.2003
(73) Patentinhaber: Fraunhofer-Gesellschaft zur Förderung der angewandten Forschung e.V., 80686 München (DE)
(72) Erfinder: BORLAK, Jürgen, 31275 Lehrte/OT Immensen (DE); THUM, Thomas, D-30177 Hannover (DE)
(74) Vertreter: Baumbach, Friedrich, Dr.
(86) Internationale Anmeldenummer: PCT/DE2001/003617
(87) Internationale Veröffentlichungsnummer: WO 2002/022865

(56) Entgegenhaltungen:
- WO-A-97/46714
- US-A- 5 130 238
- THUM T ET AL: "Gene expression in distinct regions of the heart" LANCET, XX, XX, Bd. 355, Nr. 9208, 18. März 2000 (2000-03-18), Seiten 979-983, XP004263325 ISSN: 0140-6736

## Beschreibung

Die Genexpression nimmt eine Schlüsselfunktion in der Beurteilung molekularer Vorgänge im Körper ein, und es werden große Anstrengungen unternommen, die Bedeutung der Genexpression zahlreicher Gene auch infolge der Arzneistoffwirkungen zu untersuchen und hinsichtlich ihres Prädiktionswertes bezüglich Krankheitsverlauf und Therapieerfolg zu überprüfen.

Voraussetzung für die Umsetzung einer genetischen Information ist zunächst das Umschreiben der entsprechenden DNA-Sequenz in mRNA. Die Regulation der Genexpression kann sowohl auf der Ebene der Transkription, als auch posttranskriptionell erfolgen. Für eine Evidenz-basierende Medizin ist die Aufklärung der Mechanismen, die zu einer veränderten Genexpression im Krankheitsverlauf rühren, ein wesentliches Ziel, da sich hieraus neue Therapiekonzepte ableiten, die zu einer verbesserten Behandlung von Patienten führt.

Die Analyse der Genexpression in unterschiedlichen Zelltypen und Geweben, die wichtige Hinweise über den normalen Zustand und in der Genese des pathologischen Zustandes von Zellen/Geweben erlauben, stellt in der heutigen Post-Genom-Epoche, nachdem das menschliche Genom identifiziert ist, die größte Herausforderung dar.

Eine Vielzahl von Methoden werden zur Analyse der Genexpression angewendet, u.a. Northern-Blot und RT-PCR Techniken. Mittels Chip-basierender Technologien, das sind planare Träger aus Plastik, Glas, Gelatine u.a., auf deren Oberfläche eine Vielzahl unterschiedlicher, aber in ihrer Position bekannter und adressierbarer (DNA) Moleküle angeordnet sind, können simultan Tausende von Genen hinsichtlich ihrer Expression untersucht werden.

Neben den verschiedenen methodischen Ansätzen zur Genexpressionsanalytik, werden neue Technologien entwickelt, um Nukleotidpolymorphismen (das sind unterschiedliche Varianten eines Gens) **a)** methodisch erfassen zu können und **b)** hinsichtlich ihrer biologischen Bedeutung im Krankheitsverlauf und bei medizinischen Anwendung, im Sinne einer individualisierten Therapie, beurteilen zu können.

Ein relativ junges Verfahren für fluoreszenz-basierende Genexpressions- und Genmutationsanalysen stellt die Untersuchung der mittels der PCR-Technik amplifizierten Proben in Echtzeit-PCR-Analyse-Automaten, wie im LightCycler® (Roche Diagnostics GmbH, Roche Molecular Biochemicals, Technical Note No. LC 1/99), TaqMan® (Perkin-Elmer GmbH, http://www.appliedbiosystems.com/products/ )oder anderen, dar.

Der LightCycler® ist mit einem Drei-Kanal Fluorimeter ausgestattet, das Fluoreszenz bei 530nm (SYBR®-Green), 640nm (LC-RED 640) und 705nm (LC-RED 705) detektieren kann. Für die PCR-Amplifikation im LightCycler® stellt der Hersteller Roche Diagnostics GmbH verschiedene Kits zur Verfügung, die je nach Labeling-Art (SYBR®-Green oder FRET-Verfahren; siehe unten) eingesetzt werden können.

### Farbstofflabeling

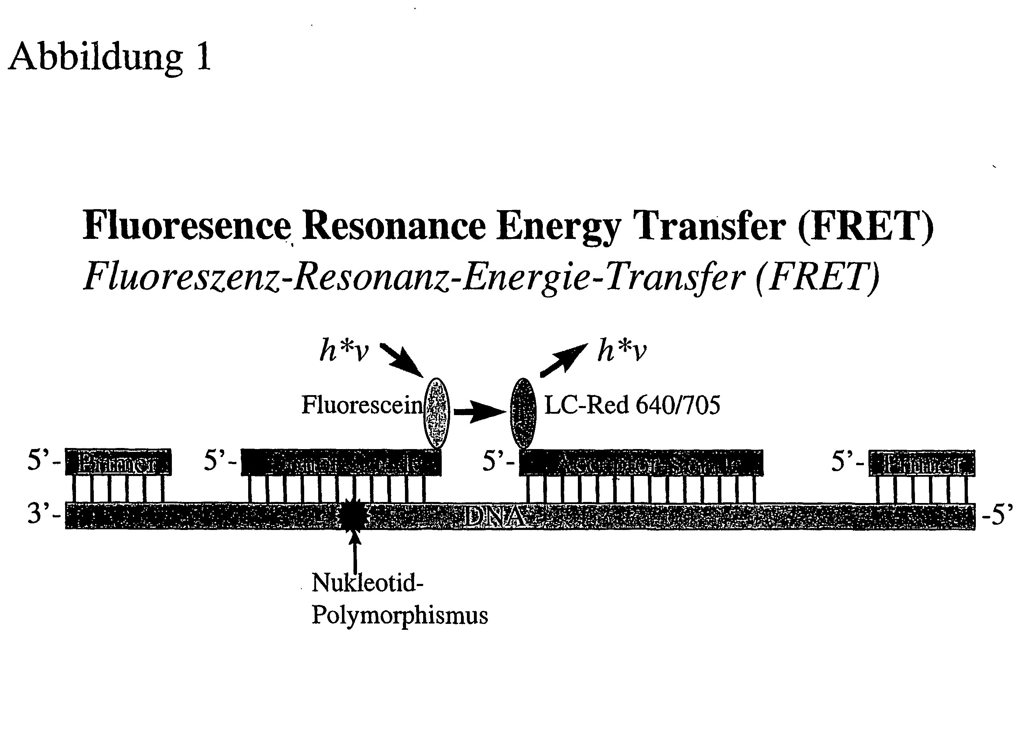

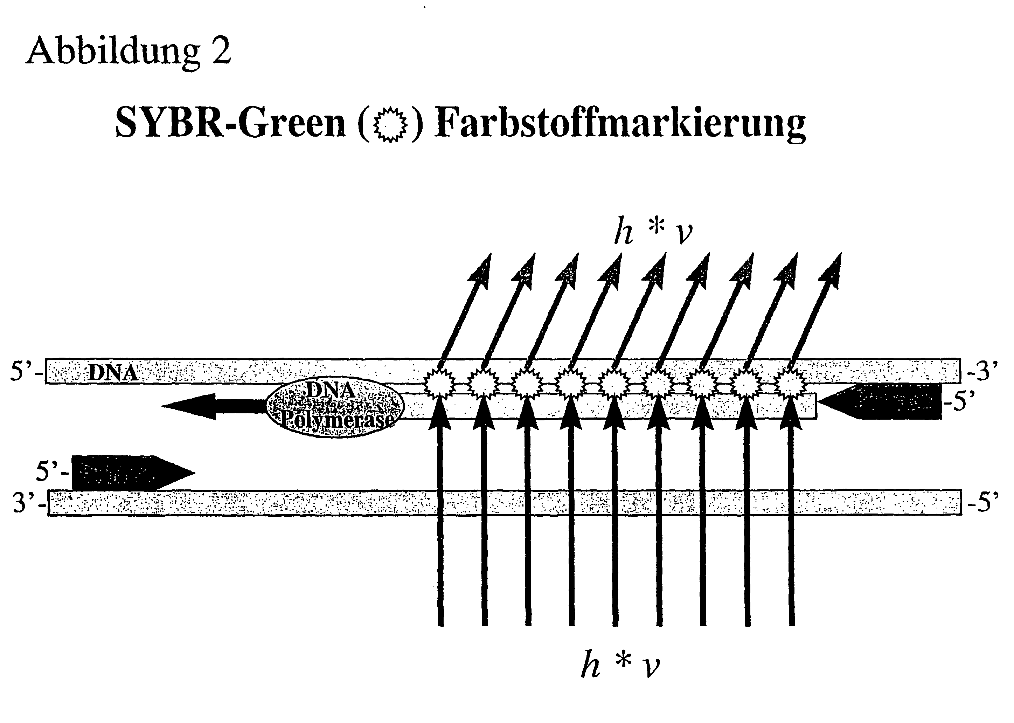

Um in der anschließenden Echtzeit PCR die jeweils de novo synthetisierte DNA vermessen zu können, muß diese durch geeignete detektierbare Farbstoffe markiert werden. Zur Detektion der Fluoreszenzsignale sind diverse Echtzeit-PCR-Detektionssysteme bekannt, mit denen es möglich ist, die gesamt PCR-Reaktion zu verfolgen. Im Folgenden werden grundsätzliche Prinzipien der Detektion erläutert:
1.) Fluoreszenz-Resonanz-Energie-Transfer (FRET)-Verfahren
   a) Als Fluoreszenz-Resonanz-Energie-Transfer (FRET) bezeichnet man einen Vorgang, bei dem ein nach Anregung durch kurzwelliges Licht fluoreszierendes Donatormolekül seine Emissionsenergie auf ein zweites Akzeptormolekül überträgt, das darauf mit der Emission längerwelligen Lichtes reagiert. Der Energietransfer von einem auf das andere Molekül erfolgt über den Elektronenfluß. Das Reportermolekül gibt dabei Auskunft über die Produktzunahme während der PCR. Das sogenannte Quenchermolekül absorbiert die Fluoreszenzsignale des Reportermoleküls, solange beide Moleküle in der Hybridisierungssonde direkt benachbart zueinander liegen. In diesem Grundzustand ist die Reporteremissionsstrahlung für den Fluoreszenzdetektor, mit dem die Produktzunahme in der PCR gemessen wird, unsichtbar. Erst mit der PCR-Produktzunahme erfolgt eine räumliche Trennung von Reporter und Quenchermolekül. Dadurch wird die Reporterfluoreszenz detektierbar und korreliert direkt mit der in der Reaktion gebildeten PCR-Produktmenge.
   b) Andere nach dem FRET-Prinzip synthetisierten Hybridisierungssonden lassen sich verwenden, um Mutationsanalysen durchzuführen. Besonders wichtig ist die Detektion sogenannter Single Nucleotide Polymorphisms (SNPs), die durch Austausch einzelner Basen zustande kommen. Zur SNP-Analyse werden zwei jeweils mit einem Fluoreszenzfarbstoff markierte Hybridisierungssonden verwendet. Eine Donorsonde bindet unmittelbar benachbart zu dem Mutationsbereich. Eine zweite Sonde wird so gefertigt, dass sie entweder komplementär zum Wildtyp oder über der Mutationsstelle bindet. In der nach der PCR durchgeführten Schmelzpunktanalyse schmilzt die Sonde bei einer bestimmten Temperatur ab. Bindet die Sonde komplementär zum Wildtyp, so schmilzt diese bei höheren Temperaturen ab, während beim Vorhandensein einer Mutation die Sonde bei niedrigeren Temperaturen abschmilzt und deshalb eine Mutationsanalyse möglich wird. Der Fluoreszenzabfall wird als negative erste Ableitung (als ein Schmelzhügel) berechnet. Durch die verschobene Schmelzkurve kann die Mutation diagnostiziert werden. Nur mittels Echt-Zeit-Detektionssystem kann dieses Verfahren mit unterschiedlichen Fluoreszenzfarbstoffen zur Anwendung kommen.
   c) Das Prinzip der Echtzeit-Detektionssysteme liegt auch dem LightCycler® von Roche Diagnostics zugrunde. Der LightCycler® hat drei Kanäle, durch die emittierte Lichtquanten von Farbstoffen detektiert werden können. DNA kann mit SYBR®-Green gelabelt werden; darüber hinaus können mit FRET-Sonden, die mit den Farbstoffen LC-Red 640 oder LC-Red 705 gelabelt sind, eingesetzt werden. Wenn das FRET-Verfahren im LightCycler® eingesetzt wird, müssen spezielle Hybridisierungssonden zum Reaktionsgemisch hinzugegeben werden, welche neben Fluorescein entweder mit LC-Red 640 oder LC-Red 705 (von Roche Diagnostics) gelabelt sind. Fluoreszenz ist nur zu beobachten, wenn beide Sonden (Donor- und Akzeptor-Sonde) in unmittelbarer räumlicher Nähe an die Zielsequenz gebunden haben. Dann kommt es zur Übertragung von Lichtquanten (h*v), dem Fluorescence Resonance Energy Transfer (FRET; siehe Abbildung 1).
   d) Beim TaqMan-Verfahren (oder 5'-Nuklease-Assay) binden die Fluoreszenz-markierten Hybridisierungssonden am komplementären Zielstrang zwischen den Primerbindungsstellen. Bei der Neustrangsynthese wird die Hybridisierungssonde durch die 5'-3'-Exonukleaseaktivität der Taq-Polymerase in kleine Fragmente geschnitten und aus dem Zielstrang freigesetzt. Reporter- und Quenchermoleküle liegen jetzt getrennt im Reaktionsmix vor, und die gemessene Zunahme der Reporterfluoreszenz pro PCR-Zyklus korreliert direkt mit der PCR-Produktzunahme.
2.) SYBR® -Green Verfahren
   a) Im SYBR®-Green Verfahren interkaliert der Farbstoff SYBR® -Green jeweils zwischen 2 komplementäre Basensträngen während der DNA Synthese und erfährt damit mit fortschreitender PCR-Reaktion einen meßbaren Fluoreszenzanstieg (siehe Abbildung 2). Bei der Verwendung von SYBR® -Green fehlt allerdings jede Spezifität hinsichtlich des zu untersuchenden Templates (das ist die DNA-Bindungsstelle), denn auch Primer-Dimere, die sich während der Reaktion bilden, verursachen einen Fluoreszenzanstieg. Dieser ist zunächst nicht von dem des gewünschten DNA-Synthese-Produktes zu unterscheiden und kann zu Fehlinterpretationen führen. Eine Differenzierung zwischen spezifischem Produkt und Primer-Dimeren kann jedoch nach abgeschlossener PCR mittels einer Schmelzkurvenanalyse erfolgen. Bei dieser werden die PCR-Produkte kontinuierlich über einen bestimmten Temperaturbereich aufgeheizt und ihrem Schmelzpunkt entsprechend nur noch als Einzelstrang vorliegen. Die damit einhergehende Fluoreszenzabnahme wird aufgezeichnet. Kleinere Fragmente, wie die Primer-Dimere weisen einen niedrigeren Schmelzpunkt auf als größere PCR-Produkte. Die daraus abgeleitete Darstellung der Temperaturabhängigkeit der Fluoreszenzsignal-Veränderungen ergibt einen Kurvenverlauf, in dem das spezifische PCR-Produkt von den Primer-Dimeren unterscheidbar wird, wenn sich die Schmelzpunkte deutlich voneinander unterscheiden.

### PCR-Amplifikation allgemein

Mit Hilfe der Polymerase-Kettenreaktion (PCR) können klar definierte DNA-Abschnitte eines Gens millionenfach vermehrt werden. Dafür werden zwei der Zielsequenz komplementäre Oligonukleotide (Primer), die sich jeweils einem der DNA-Stränge anlagern, in das PCR-Reaktionsgemisch hinzugegeben. Darüber hinaus werden ausreichende Mengen der vier Desoxynucleosidtriphosphate, eine bestimmte Menge an Magnesiumchlorid und eine hitzebeständige DNA-Polymerase in den Reaktionscocktail hinzugegeben. Es sind zahlreiche PCR-Puffersysteme bekannt. Solche Systeme für die PCR-Reaktion werden als fertige Testkits von zahlreichen Unternehmen angeboten (z.B. von Roche Diagnostics GmbH, Qiagen GmbH u.a.), wobei nur noch die spezifischen Primer und die zu untersuchende DNA hinzupipettiert werden muß.

Die Fluoreszenz-basierenden Genexpressions- und Genmutationsanalysen unter Verwendung von Reaktionsgemischen mit den an sich üblichen Komponenten und Konzentrationen bzw. unter Verwendung der kommerziell erhältlichen Testkits sind in ihrer Aussagekraft jedoch aufgrund ihrer geringeren Sensitivität, Selektivität und durch die Bildung von Primer-Dimeren sehr beschränkt einsetzbar. Insbesondere ist die Bildung von Primer-Dimeren hervorzuheben, bei denen es sich um falsche DNA-Synthese-Produkte handelt, welche den großen Nachteil haben, dass sie zu falschen Befundungen führen können, wodurch erhebliche Risiken für den Patienten und für die biomedizinische Forschung allgemein entstehen, und daher eine verläßliche Medizinaldiagnostik beispielsweise bei Begleituntersuchungen während der Therapie nicht gewährleistet werden kann. Weiterhin sind diese Untersuchungen sehr kostenintensiv, wodurch die Anzahl der möglichen Untersuchungen stark limitiert wird.

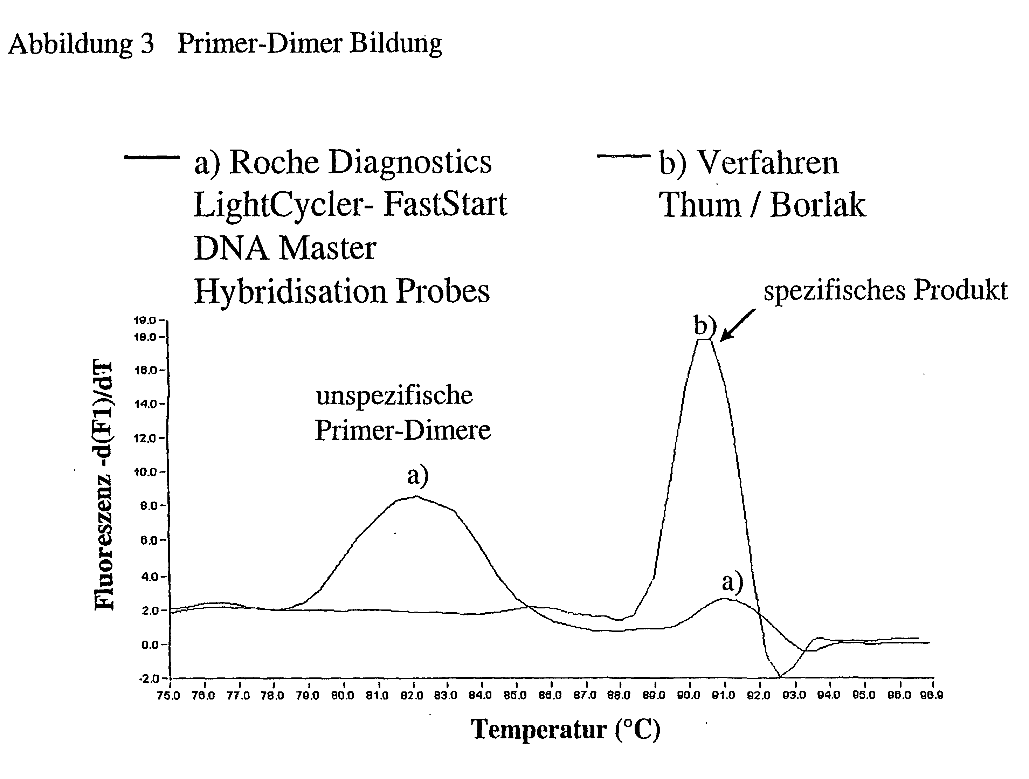

Aufgabe der Erfindung war es deshalb, Mittel zu finden und in Gemischen für die PCR-Reaktion bereitzustellen, die einerseits kostengünstig sind und die zum anderen die Selektivität und Sensitivität Fluoreszenz-basierender Genexpressions- und Genmutationsanalysen steigern sowie die Primer-Dimeren-Bildung (siehe Abbildung 3) unterdrücken und somit Fehldiagnosen und irrtümliche Befunde verhindern. Aufgabe der Erfindung war es ferner, ein Verfahren zu entwickeln, das vielfältige Untersuchungen mittels der Fluoreszenz-basierenden Genexpressions- und Genmutationsanalyse aus unterschiedlichsten Patientenprobe gestattet und sichere Befunde liefert.

Die Erfindung wird gemäß den Ansprüchen realisiert. Das erfindungsgemäße PCR-Reaktionsgemisch für Fluoreszenz-basierende Genexpressions- und Genmutationsanalysen umfasst (neben an sich üblichen Reagenzien) eine beliebige Taq-Polymerase, eine geringe Menge an BSA (Bovines Serum Albumin), welches im Reaktionsgemisch mit einer maximalen Konzentration von 400 bis 800 µg/ml vorliegt, eine hohe Menge an MgCl₂ in Abhängigkeit der eingesetzten Taq-Polymerase mit einer Konzentration von > 3,5 mM (mmol/l) bis maximal 5 mM im Reaktionsgemisch sowie ein fluoreszierendes Material. In einer bevorzugten Ausführung umfasst das PCR-Reaktionsgemisch BSA in einer Endkonzentration von 500 µg/ml, und MgCl₂ in einer Endkonzentration von 5 mM. Alle handelsüblichen Taq DNA Polymerasen können eingesetzt werden.

Es wurde festgestellt, dass bei der erfindungsgemäßen exakten Einstellung von BSA und MgCl₂ in einem an sich üblichen Reaktionsgemisch, die Echtzeit-PCR Genexpressionsund Genmutationsmessungen optimiert werden konnte. Überraschend wurden mit anderen BSA-Konzentrationen als 400 - 800 µg/ml (niedriger oder höher) keine messbaren oder nicht verwertbaren Ergebnisse erzielt.

In einer besonders bevorzugten Ausführungsvariante der Erfindung wird ein PCR-Reaktionsgemisch mit den folgenden Reaktionskomponenten bereitgestellt:

Tris-HCl, KCl, eine beliebige DNA Taq-Polymerase, dNTPs, MgCl₂, BSA und als fluoreszierende Materialien oder FRET-markierte Fluoreszenzsonden, z.B. LC-RED640 oder LC-RED705.
Für die Durchführung von HotStart-Verfahren (hierfür wird eine besondere Taq-Polymerase eingesetzt, die durch 15 minütige Erwärmung bei 95°C aktiviert wird) enthält das erfindungsgemäße Gemisch zusätzlich (NH₄)₂SO₄.

Mit dem erfindungsgemäßen Gemisch ist überraschend auch eine erhebliche Reduzierung der Menge von kostenintensivem fluoreszierenden Material verbunden, welches im Vergleich zu jetzt üblichen Kits in einer hohen Verdünnung, ca. mehr als das 15000-fache eingesetzt werden kann, vorzugsweise wird es in ca. 20.000-facher Verdünnung eingesetzt.

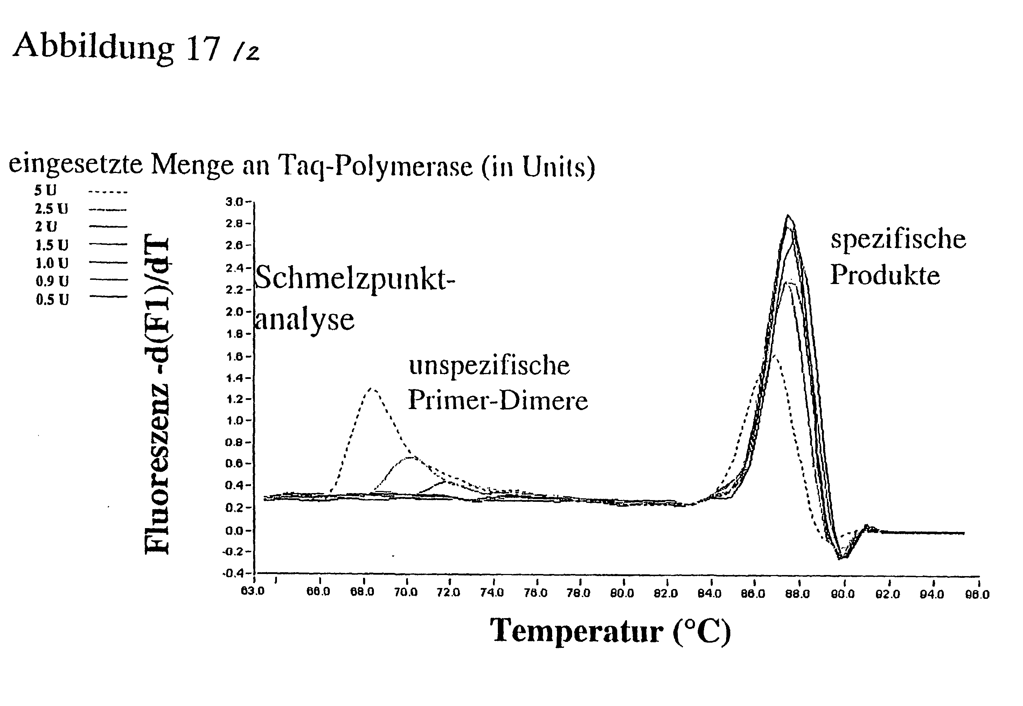

Aufgrund der verbesserten Sensitivität des erfindungsgemäßen PCR-Gemisches kann die sonst übliche Menge von 2.5Units Taq-Polymerase auf 0.9Units pro Reaktion reduziert werden, wodurch ein erhebliches Einsparungspotential entsteht. Es kann auch noch weniger als 0.9U, beispielsweise 0.5U TaqPolymerase / Reaktion, eingesetzt werden, wodurch der dynamische Amplifikationsanstieg um etwas höhere Zykluszahlen verschoben wird, hinsichtlich der Spezifität aber keine Einbußen entstehen (siehe Abb. 17).

Das erfindungsgemäße PCR-Reaktionsgemisch kann vielfältig zur Echtzeit-PCR mittels einer Fluoreszenz-basierenden Genexpressions- und Genmutationsanalyse verwendet werden. Dabei werden nach an sich fachgemäßen Methoden DNA oder RNA aus beliebigen biologischen Materialien isoliert und, ggf. nach vorheriger Bearbeitung, wie z.B. Umschreibung in cDNA, mit spezifischen Oligonukleotidprimern (deren Herstellung und Auffinden dem Fachmann bekannt ist) in Kontakt gebracht. Anschließend erfolgt in Abhängigkeit des Farbstoff-Labelings oder der eingesetzten Sonden die Auswertung, vorzugsweise nach dem SYBR®-Green- oder nach dem FRET-Verfahren.

Zur Auswertung werden handelsübliche Echtzeit-PCR-Analyse-Automaten eingesetzt, wie z.B. ein LightCycler® oder TaqMan®. Die vorliegende PCR-Reagenzmischung ist zum Nachweis zahlreicher und vielfältiger Untersuchungen mittels der Flüoreszenz-basierenden Genexpressions- und Genmutationsanalyse aus unterschiedlichsten Patientenprobe geeignet und liefert wie anhand von Ausführungsbeispielen belegt wird, sichere Befunde.

So erfolgte z.B. der Nachweis einer Genpolymorphie in Morbus Meulengracht Patienten, der Nachweis der sarcoplasmatischen Calcium-ATPase aus gewonnener cDNA von humanen Herzen, der Nachweis des beta-myosin-heavy-chain- (MHC) Gens aus gewonnener cDNA von humanem Herzgewebe, der Nachweis des brain natriuretic peptide (BNP) Gens aus gewonnener cDNA von humanem Herzgewebe, der Nachweis des atrialen natriuretischen Peptid (ANP) Gens aus cDNA von kultivierten Kardiomyozyten, der Nachweis des alpha skelettalen Aktin Gens aus cDNA von kultivierten Kardiomyozyten, der Nachweis des Albumin Gens aus cDNA von kultivierten Hepatozyten, der Nachweis des Transkriptionsfaktors HNF-3gamma aus cDNA von kultivierten Hepatozyten, der Nachweis des N-Acetyltransferase 2 Allel 5* Genotyps aus humaner lymphozytärer DNA.

Die Verwendung des erfindungsgemäßen PCR-Reaktionsgemisches führte zu einer deutlich verbesserten Sensitivität und Selektivität von Fluoreszenz-basierenden Genexpressions- und Genmutationsanalysen im tierischen, bakteriellen, pflanzlichen und menschlichen Genom und verhinderte Fehldiagnosen. Insbesondere ist hervorzuheben, dass es möglich ist, derartige Nachweise bzw. Untersuchungen an Proben durchzuführen, die wegen ihrer geringen RNA- bzw. DNA-Konzentration einer entsprechenden Analyse vorher nicht zugänglich waren. Das erfindungsgemäße PCR-Reaktionsgemisch ermöglicht eine deutliche Steigerung der Aussagekraft der semi- und totalquantitativen Ermittlung der Genexpression in Geweben und Organen im gesunden, erkrankten und medikamentös beeinflußten Zustand.

Darüber hinaus konnten die Kosten für die Durchführung einer Analyse aufgrund der eingesetzten billigen Substanzen und erheblichen Minimierung der Menge an Fluoreszenzmittel enorm gesenkt werden. Die Kosten pro Probe im Vergleich zu handelsüblichen Tests für eine Genexpressionsanalyse oder zum Nachweis von Nukleotidpolymorphismen konnten bis zu 1/6 (im Falle des HotStart-Verfahrens) bis zu 1/40 (Standard-Verfahren) des ursprünglichen Preises reduziert werden.

Das technisches Anwendungsgebiet der Erfindung umfaßt vor allem **a)** die Pharmakogenomics und hier insbesondere die Entdeckung genomischer Targets für in der Forschung und Entwicklung befindlichen Arzneistoffkandidaten, oder bereits auf dem Markt eingeführter Produkte, **b)** den Nachweis von Nukleotid-Polymorphismen, insbesondere in der molekularen Diagnostik von Erkrankungen basierend auf Gen-Mutationsanalysen und Gen-Polymorphien, in der Arzneimitteltherapie und hier insbesondere in der individualisierten Dosierung von Arzneistoffen und zur rationalen Interpretation pharmakokinetischer Therapieverläufe, **c)** für die Charakterisierung von potentiellen Arzneistoffen auf der Genexpressionsebene, **d)** die Toxikogenomics und hier insbesondere den Einsatz bei toxikologischen Untersuchungen in der präklinischen Entwicklung und zur Vorhersage von toxischen Wirkungen und zur toxikologischen Charakterisierung von einzelnen Stoffen und Stoffgemischen auf der Genexpressions-Ebene,
e) die molekulare Diagnostik und hier insbesondere das Screening und die Diagnose von krankheitsrelevanten Genen, das Monitoring im Krankheits- und Therapie-Verlauf und die molekulare Prognostik von Erkrankungen und **f)** die Forschung und hier insbesondere die Erkennung von molekularen Interaktionen von Stoffen, Stoffgemischen und biologischen Agenzien auf der Genomebene, die Erkennung von Gen-Interaktionen und Funktionsanalyse neuer Gene, einschließlich Sequenzanalysen und Genklonierungen.

### Ausführungsbeispiele

Die Nachweise erfolgten mit einem PCR-Reaktionsgemisch, das sich aus den folgenden Komponenten zusammensetzt:

### Standard-Verfahren

10mM Tris-Cl, 50mM KCl, 0.9Units einer beliebigen DNA Taq-Polymerase, 200µM dNTPs, 5mM MgCl₂, 500µg/ml BSA und als fluoreszierende Materialien SYBR®-Green I (1:20000 verdünnt) oder FRET-markierte Fluoreszenzsonden, z.B. LC-RED640 oder LC-RED705.

### HotStart-Verfahren

- HotStarTaq Master Mix Kit der Qiagen GmbH gemäß HotStarTaq PCR Handbook von 2001 (HotStarTaq Polymerase, die Puffer Tris-Cl, KCl und Ammoniumsulfat, pH 8,7, 1,5 mM MgCl₂, 200 µM dNTPs)
- zusätzlich BSA (500 µg/l), MgCl₂ für eine Endkonzentration von 5 mM,
- SYBR®-Green I in einer 20000-fachen Verdünnung oder für FRET-Verfahren Hybridisierungssonden (Donor-Sonde mit Fluoreszein gelabelt, Acceptor-Sonde mit LC-RED 640 bzw. mit LC-RED705)

### Probengewinnung und Nukleinsäuren-Isolation

Zuerst wird das zu untersuchende biologische Material in geeigneter Weise gewonnen und isoliert. Demnach werden Nukleinsäuren (RNA und DNA) aus Vollblut (kernhaltige Lymphozyten) oder tierischen / menschlichen Geweben isoliert und aufgereinigt.
Mittels Standardisierter Methoden kann DNA oder RNA aus Blut und/oder Geweben isoliert werden. Im Falle der RNA-Gewinnung muß diese vor Einsatz in der PCR in copyDNA umgeschrieben werden, um darauffolgend große Mengen der Ziel-DNA synthetisieren zu können. Dazu wird eine definierte Menge an RNA mittels reverser Transcriptase in copyDNA umgeschrieben.

### Beispiel 1)

### Nachweis einer Genpolymorphie in Morbus Meulengracht Patienten mittels FRET-Verfahren

Der Morbus Meulengracht (Gilberts syndrome) ist eine Erkrankung, die durch eine TA-Insertions-Polymorphie im Bereich der TATA-Box des Uridin-5'-diphosphoglukose-Glukuronyltransferase-Gens (UGT1A1) hervorgerufen wird. Erfindungsgemäß wurde eine Methode entwickelt, um diese Polymorphie schnell und einfach zu diagnostizieren.

Nach der Entnahme von Blutproben (200µl Vollblut pro Patient in der Regel in EDTA haltigen Gefäßen, z.B. Monovetten) werden diese bei -20° bis zur Probenaufarbeitung gelagert. Mittels eines DNA Isolations Kits wird die DNA aus den nukleierten Blutzellen isoliert und aufgereinigt. In die gekühlte LightCycler Kapillare wird vergleichend hinzugegeben:
a) ein Standard PCR-Reaktionsgemisch der Roche Diagnostics GmbH (LightCycler DNA Master Hybridization Probes oder LightCycler FastStartDNA Master Hybridization Probes.
b) das erfindungsgemäße PCR-Reaktionsgemisch wie oben erwähnt

Beiden Gemischen werden spezifische Oligonukleotidprimer (je 400nM), die Hybridisierungssonden (je 100nM; Donor-Sonde mit Fluoreszein gelabelt, Acceptor-Sonde mit LC-RED 640) und die patientenspezifische DNA (2µl) hinzupipettiert.

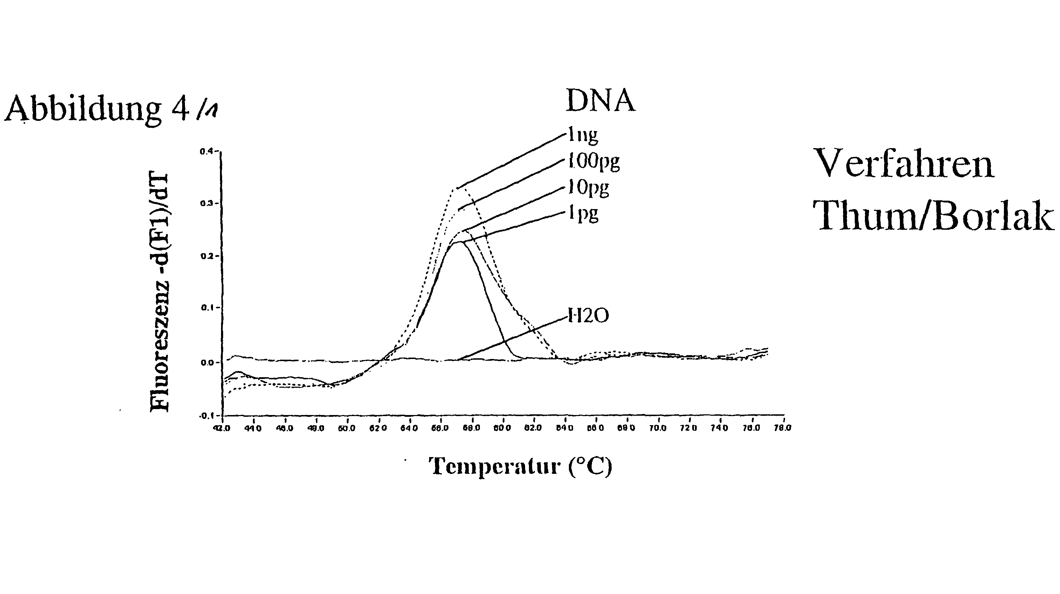

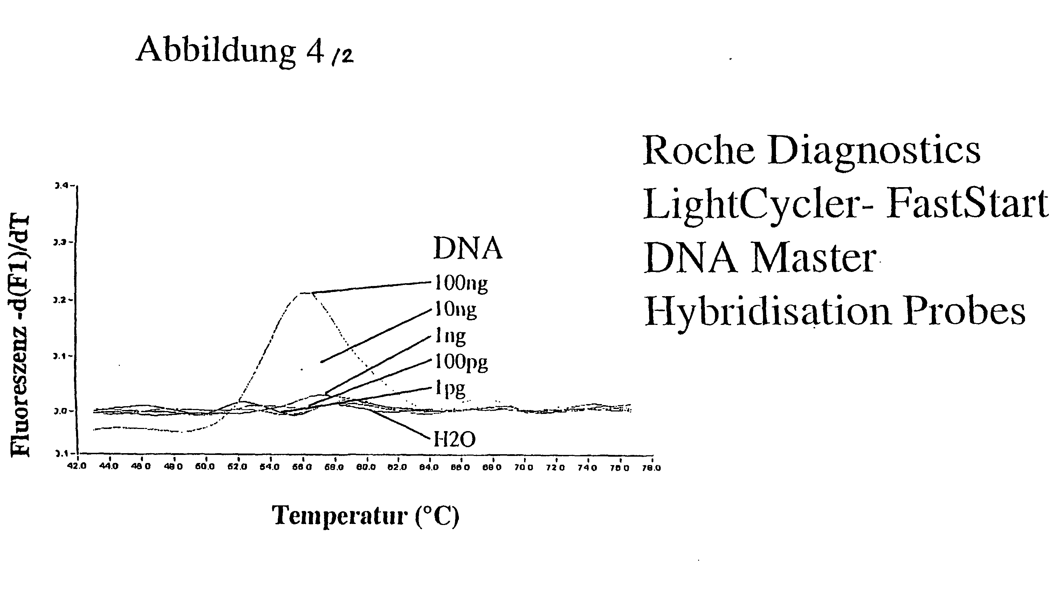

Die PCR-Reaktion wird dann mit einer 15 minütigen Denaturierungsphase bei 95°C gestartet. (Im Falle des Lightcycler- DNA Master Hybridisation-Kits betrug die Denaturierungsphase 2min). Danach werden 50 Zyklen mit folgendem Ablauf wiederholt: 95°C für 3s, dann 55°C für 7s und 72°C für 12s. Im Anschluss an die PCR-Reaktion wird eine Schmelzpunktanalyse durchgeführt. Dazu werden die entstandenen Syntheseprodukte auf 45°C erwärmt und langsam (0,2°C/s) auf 80°C erhitzt. Während dieser Zeitspanne wird online/Echtzeit die Fluoreszenz ermittelt.
Sobald die über der zu detektierenden Mutation liegende Hybridisierungssonde abschmilzt, findet kein Fluoresence Resonance Energy Transfer (FRET siehe oben) mehr statt. Die Fluoreszenz wird durch eine spezielle Software anhand der Schmelzkurven berechnet, welche dann als erste negative Ableitung in Abhängigkeit von der Temperatur (-dF/dT vs T) graphisch angegeben werden (siehe Abbildung 4).

Die eingesetzten Mittel sind der Tabelle 1 zu entnehmen. Im vorliegenden Anwendungsbeispiel wird die Sensitivität des erfindungsgemäßen FRET-Reaktionsgemisches mit dem von Roche Diagnostics GmbH (Lightcycler- FastStart DNA Master Hybridisation Probes) verglichen.

Wie der Abbildung 4 zu entnehmen ist, ist das erfindungsgemäße PCR-Reaktionsgemisch bis zu 10.000fach im Vergleich zu den angebotenen Kits von Roche Diagnostics sensitiver. Darüber hinaus ist ein ausgesprochen wichtiges Merkmal, dass die Schmelzkurven bei der Verwendung des erfindungsgemäßen Kits höher und schmaler sind, womit spezifischer und somit eindeutiger die einzelnen Genotypen unterschieden werden können. Für die medizinische Beurteilung von Nukleotidpolymorphismen, in der medizinischen Diagnostik und für die individualisierte dosisangepaßte Therapie ist dieser Unterschied von grundsätzlicher Bedeutung (siehe Abbildung 4).

### Beispiel 2)

### Nachweis der sarcoplasmatischen Calcium-ATPase aus cDNA des humanen Herzens mittels SYBR®-Green-Labelings

Nach Explantation des humanen Herzens im Rahmen einer Herztransplantation wurde einem Patienten Biopsiematerial entnommen und unmittelbar in flüssigem Stickstoff bis zur Weiterverarbeitung eingefroren. Dann wurden 50mg Herzgewebe entnommen und mittels standardisierter Verfahren wurde RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare wiederum vergleichend hinzugegeben
a) ein PCR-Reaktionsgemisch (LightCycler- DNA Master SYBR®-Green I oder Lightcycler- FastStart DNA Master SYBR®-Green I (beide Roche Diagnostics GmbH) oder
b) das erfindungsgemäße PCR-Gemisch wie oben erwähnt

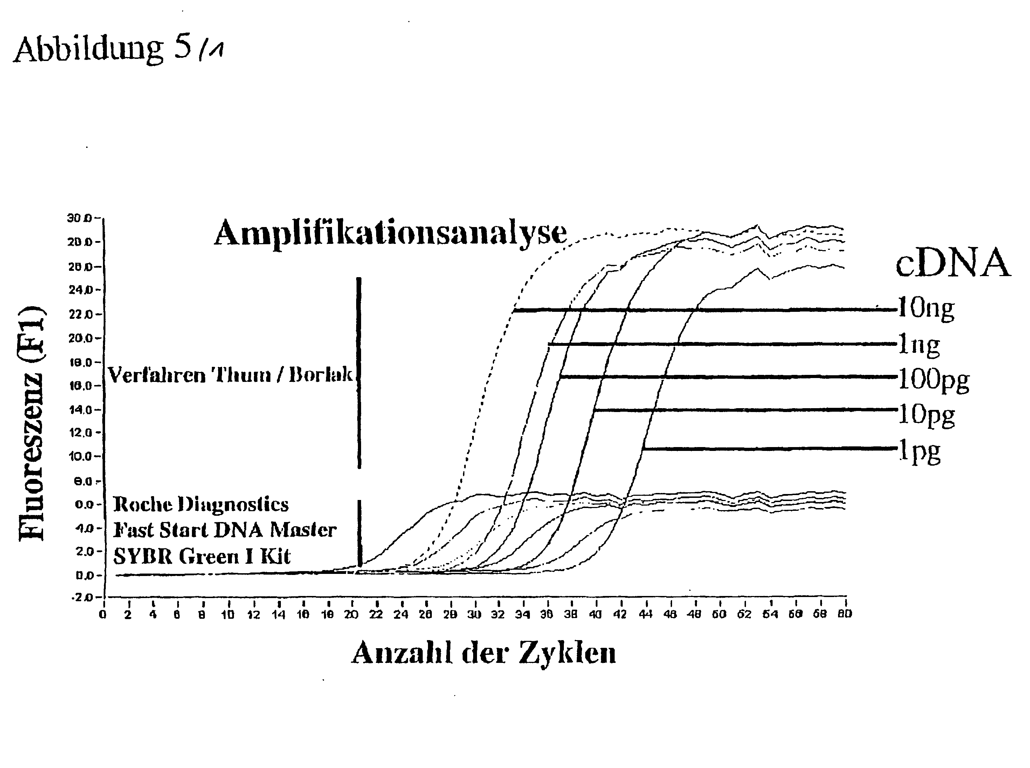

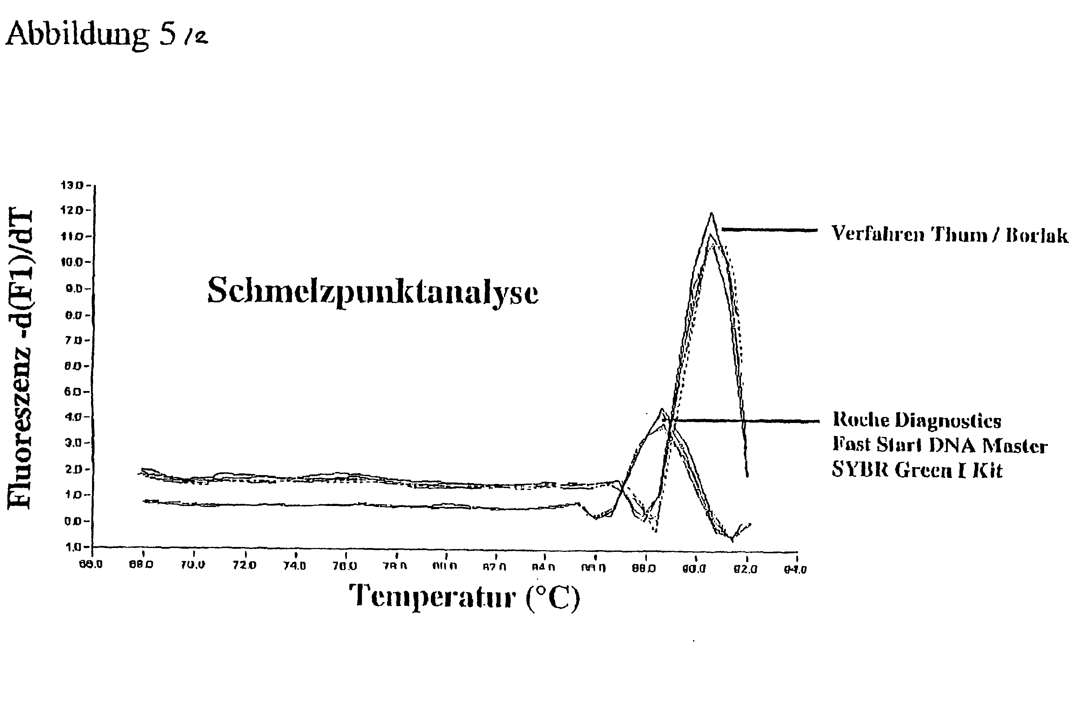

Dazu werden je 400nM der spezifischen Oligonukleotid Primer und die entsprechende copyDNA hinzupipettiert. Die PCR-Reaktion wird im Anschluss an eine 15 minütige Denaturierungsphase bei 95°C gestartet (Im Falle des Lightcycler- DNA Master SYBR®-Green I-Kits betrug die Denaturierungsphase 2min). Danach werden 60 Zyklen mit folgendem Ablauf wiederholt: 95°C für 3s, dann 55°C für 7s und 72°C für 12s. Bei 87°C erfolgte bei jedem Zyklus pro Kapillare eine aktuelle Fluoreszenzmessung, aus der sich die in Abbildung 5 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt. Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 5).

Die eingesetzten Mittel sind der Tabelle 1 zu entnehmen. In diesem Anwendungsbeispiel wird die Sensitivität des beanspruchten SYBR®-Green Reaktionsgemisches mit den AnalyseKits von Roche Diagnostics GmbH verglichen. Abbildung 5 zeigt, dass die Verwendung des erfindungsgemäßen Gemisches zu einer wesentlich verbesserten, weil höheren Dynamik (Fluoreszenzanstieg / Zyklus) bei der DNA-Synthese führt. Auch bei der Schmelzpunktanalyse ist die Dynamik (Fluoreszenzverlust / sec) viel höher, so dass die Schmelzpunktpeaks schmaler und die Amplitude höher ist, wodurch die Genexpression spezifischer und somit genauer ermittelt werden kann.

In den folgenden Beispielen ist die erfolgreiche Verwendung des neuen Reaktionsgemisches bei der Amplifikation krankeitsrelevanter Gene aus humanen und tierischen Geweben und in Zellkulturexperimenten beschrieben.

### Beispiel 3)

### Nachweis des beta-myosin-heavy-chain (MHC)-Gens aus cDNA des humanen Herzens mittels SYBR®-Green-Labelings

Nach Explantation des humanen Herzens im Rahmen einer Herztransplantation wurde Biopsiematerial entnommen und unmittelbar in flüssigem Stickstoff bis zur Weiterverarbeitung eingefroren. Dann wurden 50mg Herzgewebe entnommen und mittels standardisierter Verfahren wurde RNA isoliert und aufgereinigt. 2µg RNA und Random-Primer (Roche Diagnostics GmbH) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), RNase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng, 1ng und 100pg).

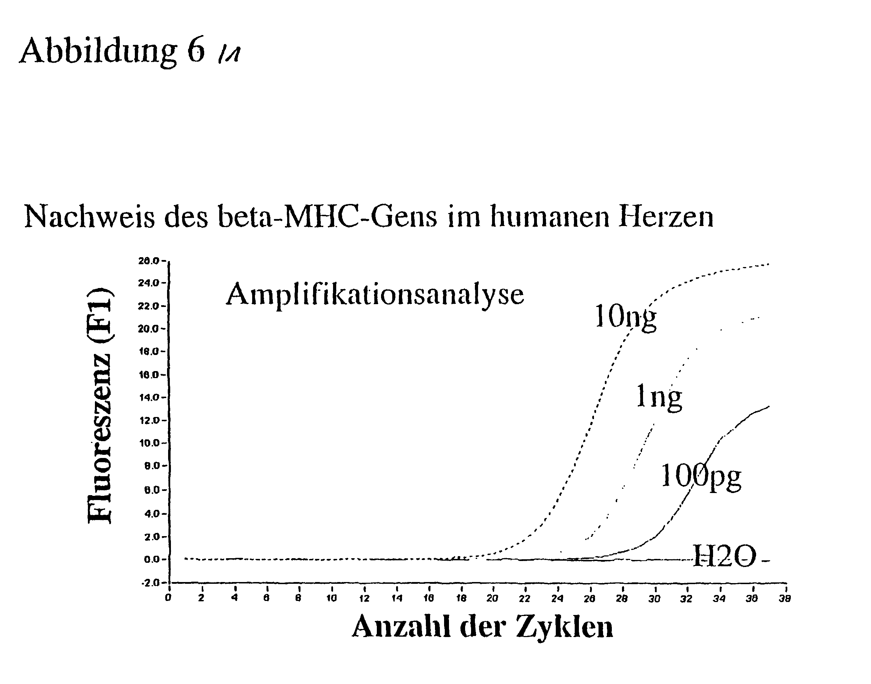

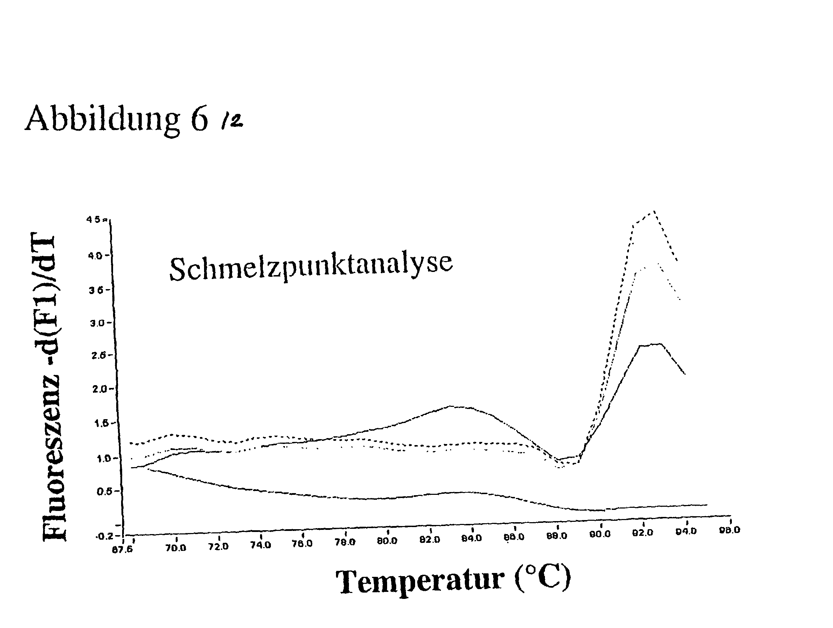

Die PCR-Reaktion wird im Anschluss an eine 15 minütige Denaturierungsphase bei 95°C gestartet. Danach werden 38 Zyklen mit folgendem Ablauf wiederholt: 95°C für 3s, dann 57°C für 8s und 72°C für 12s. Bei 89°C erfolgte bei jedem Zyklus pro Kapillare eine aktuelle Fluoreszenzmessung, aus der sich die in Abbildung 6 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt. Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 6).

### Beispiel 4)

### Nachweis des brain natriuretic peptide (BNP) -Gens aus cDNA des humanen Herzens mittels SYBR®-Green-Labelings

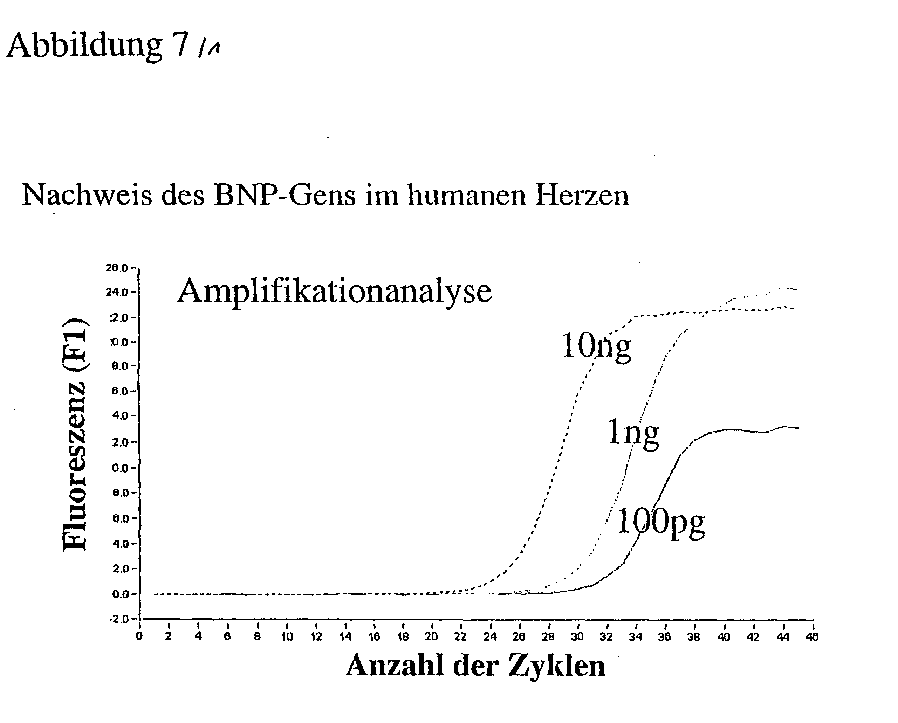

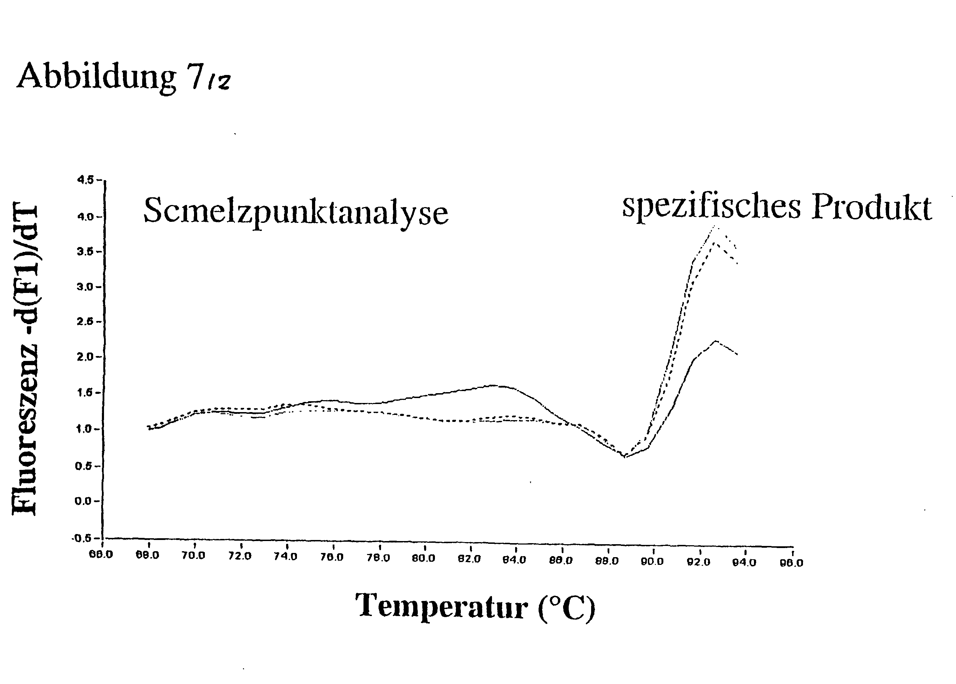

Nach Explantation des humanen Herzens im Rahmen einer Herztransplantation wurde Biopsiematerial entnommen und unmittelbar in flüssigem Stickstoff bis zur Weiterverarbeitung eingefroren. Dann wurden 50mg Herzgewebe entnommen und mittels standardisierter Verfahren wurde RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng, 1ng und 100pg).
Die PCR-Reaktion wird im Anschluss an eine 15 minütige Denaturierungsphase bei 95°C gestartet. Danach werden 46 Zyklen mit folgendem Ablauf wiederholt: 95°C für 3s, dann 55°C für 8s und 72°C für 10s. Bei 89°C erfolgte bei jedem Zyklus pro Kapillare eine aktuelle Fluoreszenzmessung, aus der sich die in Abbildung 7 dargestellte Echtzeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt.
Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 7).

### Beispiel 5)

### Nachweis des atrialen natriuretischen Peptids (ANP) Gens aus cDNA kultivierter Kardiomyozyten der Ratte mittels SYBR®-Green Technik

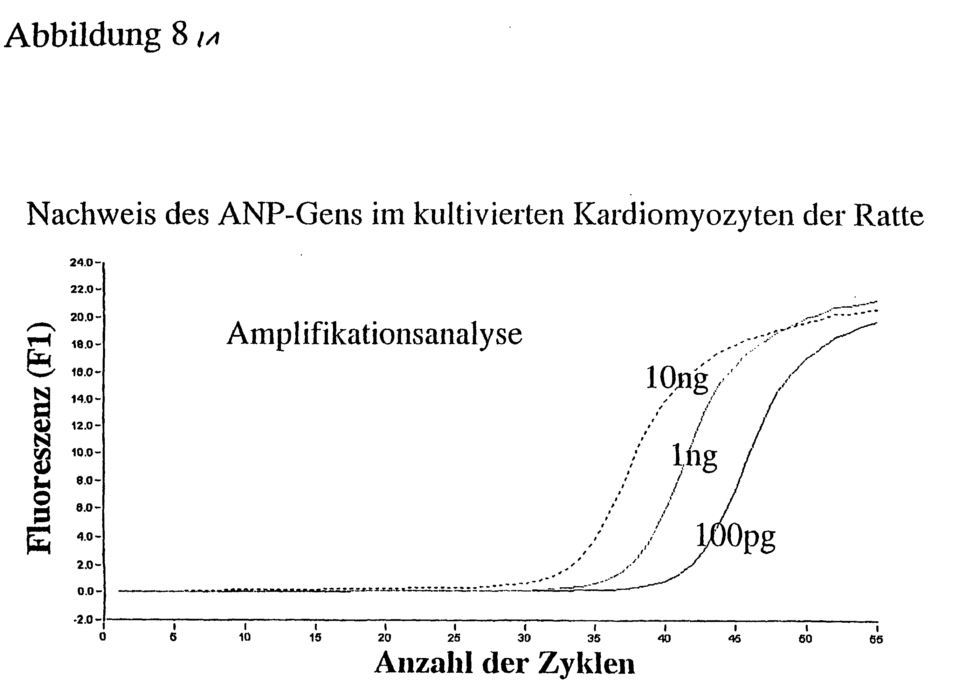

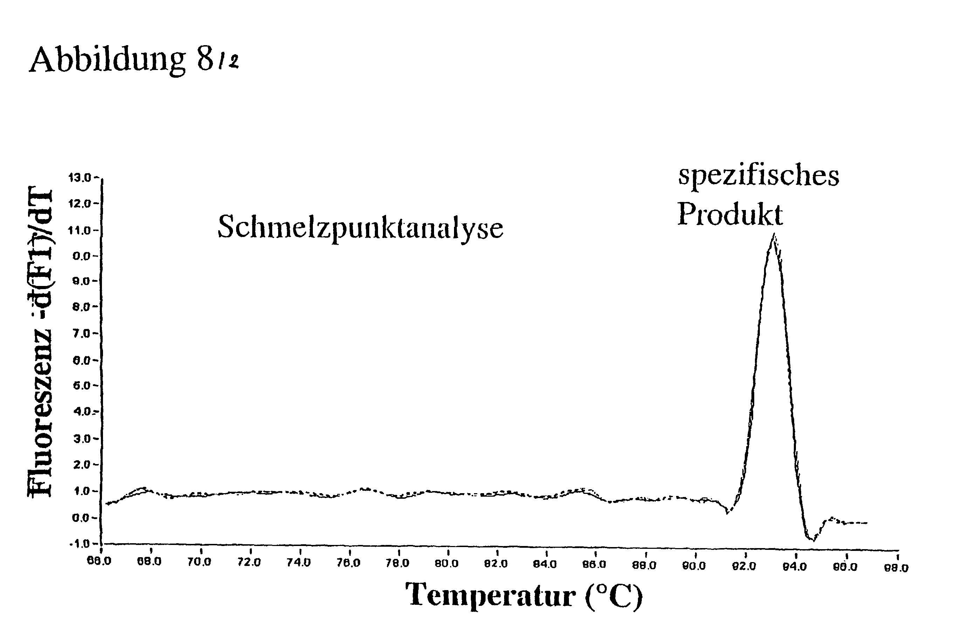

Nach Isolation und 48h Kultur adulter Kardiomyozyten der Ratte wurden die Zellen geerntet und unmittelbar in flüssigem Stickstoff bis zur Weiterverarbeitung eingefroren. Mittels standardisierter Verfahren wurde RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng, 1ng und 100pg).
Die PCR-Reaktion wird im Anschluss an eine 15 minütige Denaturierungsphase bei 95°C gestartet. Danach werden 55 Zyklen mit folgendem Ablauf wiederholt: 95°C für 3s, dann 55°C für 7s und 72°C für 12s. Bei 91°C erfolgte bei jedem Zyklus pro Kapillare eine aktuelle Fluoreszenzmessung, aus der sich die in Abbildung 8 dargestellte Echtzeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt. Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 8).

### Beispiel 6)

### Nachweis des alpha skeletalen actin Gens aus cDNA von frisch entnommenen Herzgewebe der Ratte mittels SYBR®-Green Technik

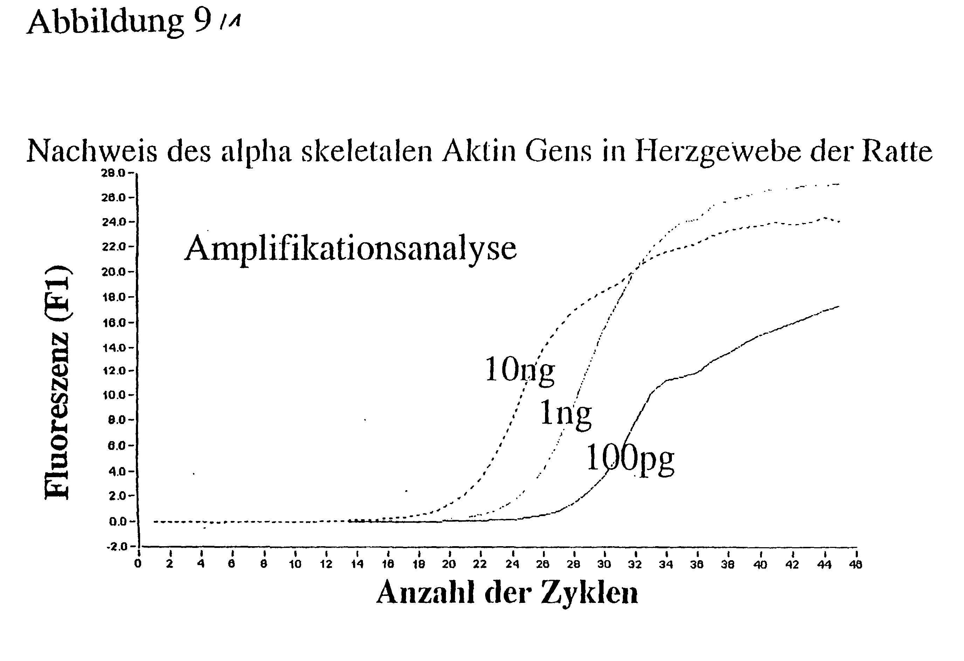

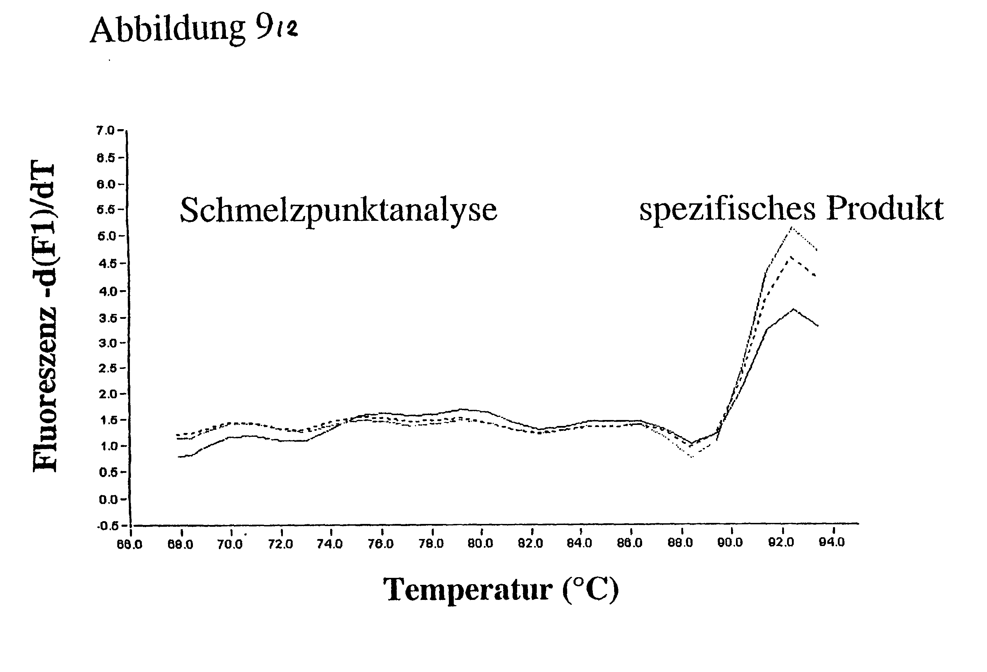

Nach Explantation des Rattenherzens wurde Biopsiematerial entnommen und unmittelbar in flüssigem Stickstoff bis zur Weiterverarbeitung eingefroren. Mittels standardisierter Verfahren wurde RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primem und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng, 1ng und 100pg).
Die PCR-Reaktion wird im Anschluss an eine 15 minütige Denaturierungsphase bei 95°C gestartet. Danach werden 46 Zyklen mit folgendem Ablauf wiederholt: 95°C für 3s, dann 55°C für 7s und 72°C für 15s. Bei 90°C erfolgte bei jedem Zyklus pro Kapillare eine aktuelle Fluoreszenzmessung, aus der sich die in Abbildung 9 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt. Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert.
Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 9).

### Beispiel 7)

### Nachweis des Albumin Gens aus cDNA von kultivierten Hepatozyten der Ratte mittels SYBR®-Green Technik

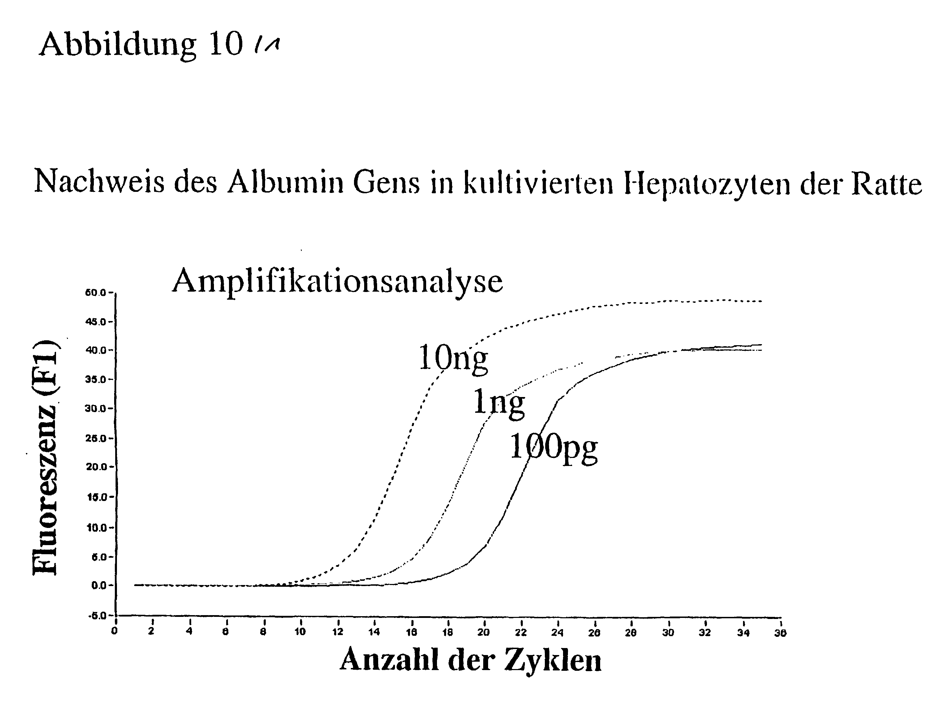

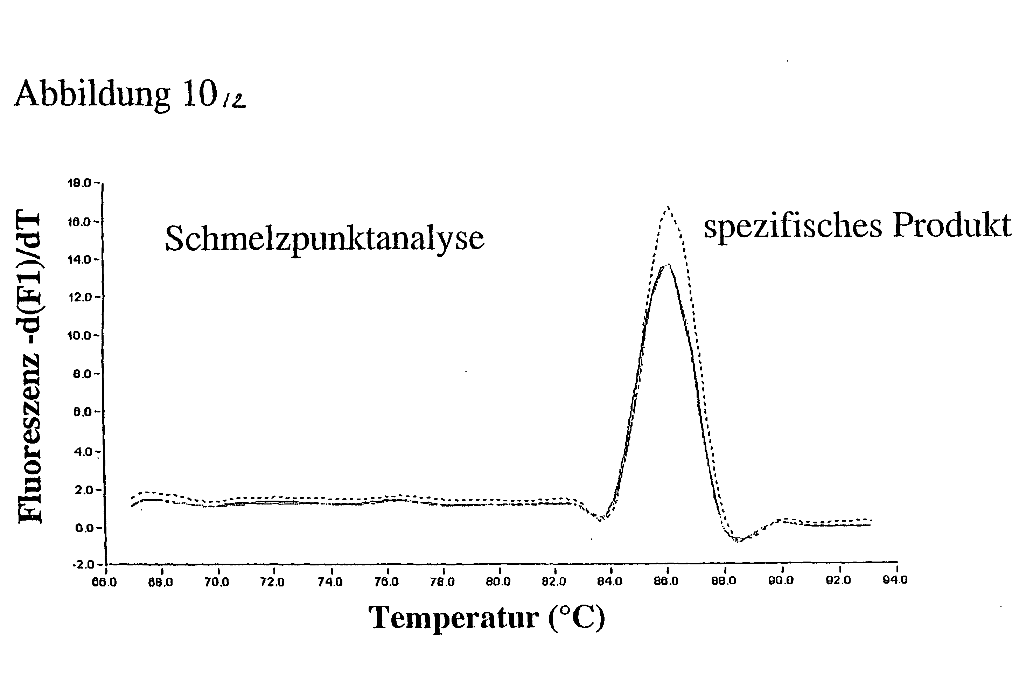

Nach Isolation und 48h Kultivierung von Hepatozyten der Ratte wurden die Zellen geerntet und mittels standardisierter Verfahren wurde RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng, 1ng und 100pg).
Die PCR-Reaktion wird im Anschluss an eine 15 minütige Denaturierungsphase bei 95°C gestartet. Danach werden 36 Zyklen mit folgendem Ablauf wiederholt: 95°C für 3s, dann 55°C für 7s und 72°C für 12s. Bei 83°C erfolgte bei jedem Zyklus pro Kapillare eine aktuelle Fluoreszenzmessung, aus der sich die in Abbildung 10 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 68°C erwärmt und langsam (0,2°C/s) auf 95°C erhitzt. Während dieser Zeitspanne wird online die Fluoreszenz ermittelt. Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 10).

### Beispiel 8)

### Nachweis des Transkriptionsfaktors Hepatic Nuclear Factor (HNF) 3 gamma aus cDNA kultivierter Hepatozyten der Ratte mittels SYBR®-Green Technik

Nach Isolation und 48h Kultivierung von Hepatozyten der Ratte wurden die Zellen geerntet und es wurde mittels standardisierter Verfahren RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.

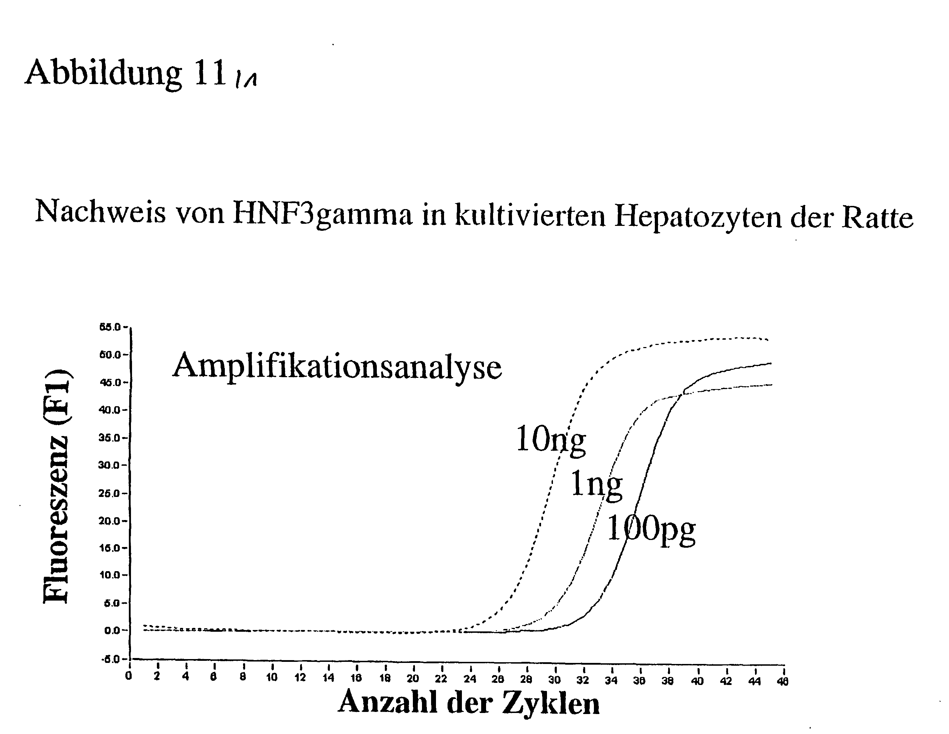

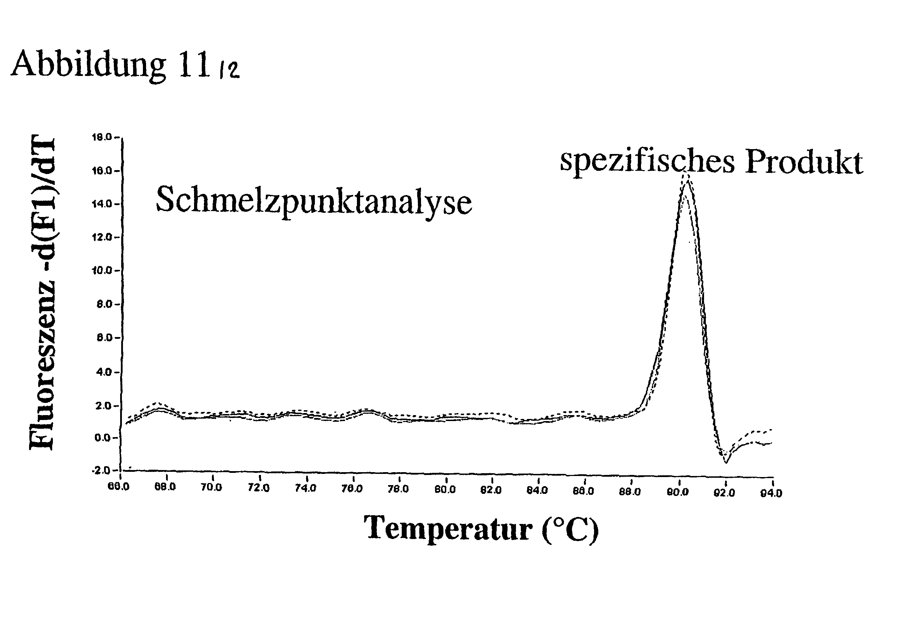

Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (6 mal 100pg).
Die PCR-Reaktion wird im Anschluss an eine 15 minütige Denaturierungsphase bei 95°C gestartet. Danach werden 46 Zyklen mit folgendem Ablauf wiederholt: 95°C für 3s, dann 57°C für 8s und 72°C für 12s. Bei 88°C erfolgte bei jedem Zyklus pro Kapillare eine aktuelle Fluoreszenzmessung, aus der sich die in Abbildung 11 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 66°C erwärmt und langsam (0,2°C/s) auf 95°C erhitzt. Während dieser Zeitspanne wird online die Fluoreszenz ermittelt.
Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 11).

### Beispiel 9)

### Identifikation des N-Acetyltransferase 2 Allel5* Genotyps aus humaner lymphozytärer DNA mittels FRET-Verfahren mittels FERT-Technik

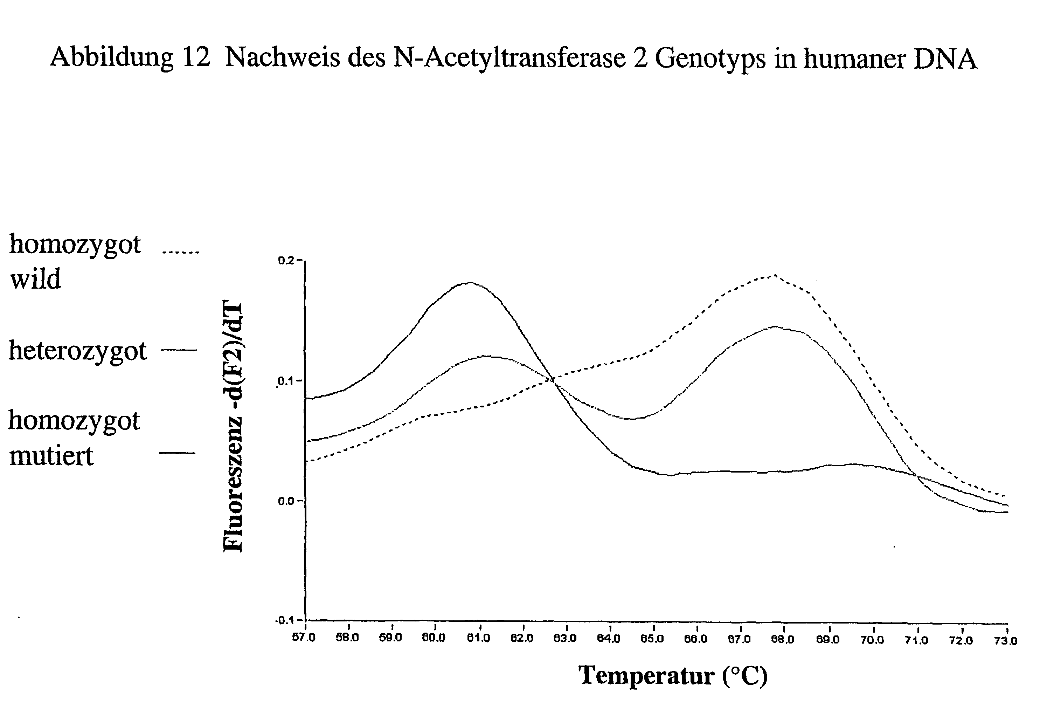

Die N-Acetyltransferase 2 ist in der Verstoffwechselung vieler Arzneimittel involviert. Patienten mit Polymorphismen (Genmutationen durch Basenaustaüsch oder Deletionen) haben ein erhöhtes Risiko durch Arzneistoffnebenwirkungen vergiftet zu werden.
Nach der Entnahme von Blutproben (200µl Vollblut pro Patient in der Regel in EDTA haltigen Gefäßen, z.B. Monovetten) werden diese bei -20° bis zur Probenaufarbeitung gelagert. Mittels eines DNA Isolations Kits wird die DNA aus den nukleierten Blutzellen isoliert und aufgereinigt. In die gekühlte LightCycler Kapillare wird das beanspruchte PCR-Reaktionsgemisch (siehe Tabelle 1) hinzugegeben. Dazu werden spezifische Oligonukleotidprimer (je 400nM), die Hybridisierungssonden (je 10nM; Donor-Sonde mit Fluoreszein gelabelt, Acceptor-Sonde mit LC-RED 640) und die patientenspezifische DNA (2µl) hinzupipettiert.
Die PCR-Reaktion wird dann mit einer 15 minütigen Denaturierungsphase bei 95°C gestartet. (Im Falle des Lightcycler- DNA Master Hybridisation-Kits betrug die Denaturierungsphase 2min). Danach werden 60 Zyklen mit folgendem Ablauf wiederholt: 95°C für 3s, dann 45°C für 10s und 72°C für 20s. Im Anschluss an die PCR-Reaktion wird eine Schmelzpunktanalyse durchgeführt. Dazu werden die entstandenen Syntheseprodukte auf 45°C erwärmt und langsam (0,2°C/s) auf 75°C erhitzt. Während dieser Zeitspanne wird online/Echtzeit die Fluoreszenz ermittelt. Sobald die über der zu detektierenden Mutation liegende Hybridisierungssonde abschmilzt, findet kein Fluorescence Resonance Energy Transfer (siehe oben) mehr statt. Die Fluoreszenz wird durch eine spezielle Software anhand der Schmelzkurven berechnet, welche dann als erste negative Ableitung in Abhängigkeit von der Temperatur (-dF/dT vs T) graphisch angegeben werden (siehe Abbildung 12).

### Beispiel 10)

### Nachweis des CYP2J3 Gens aus cDNA explantierter Herzen der Ratte mittels SYBR®-Green Technik

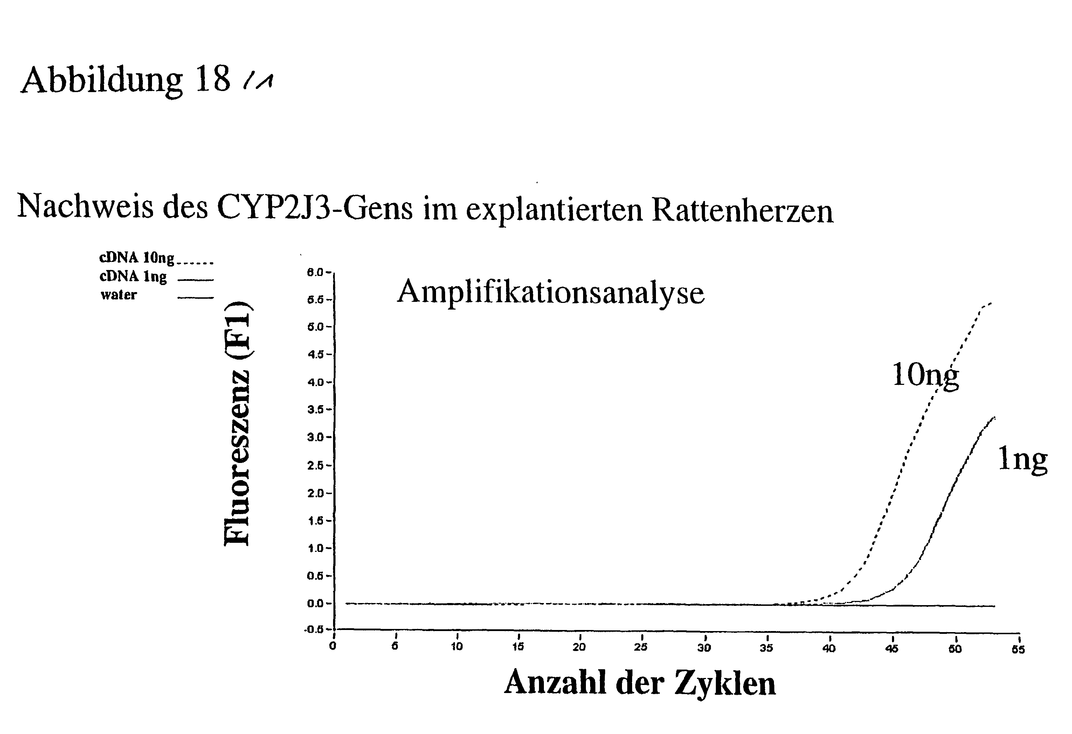

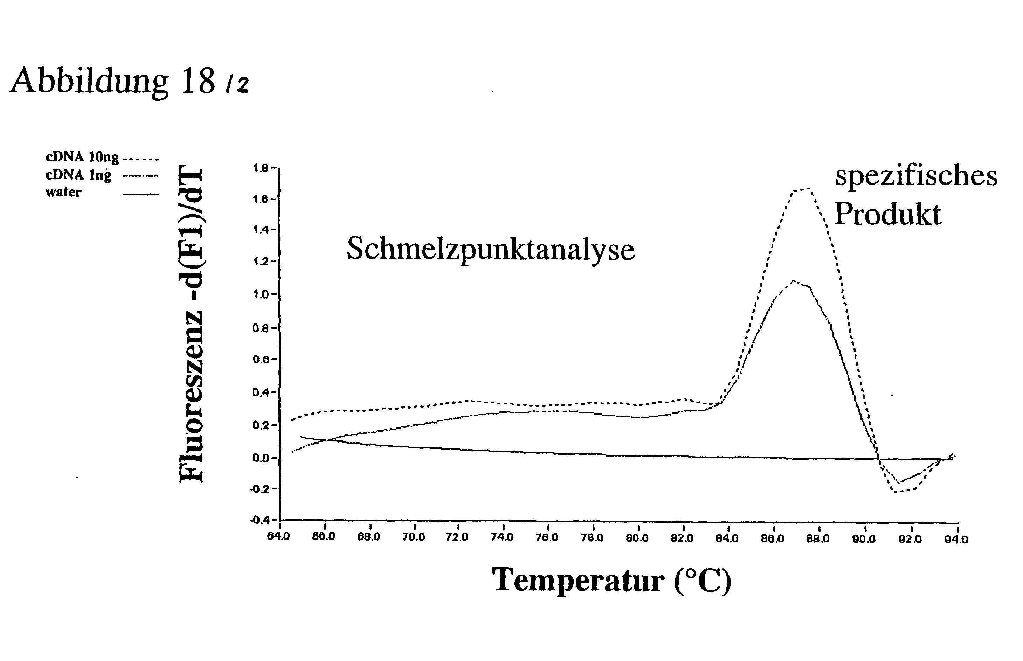

Nach Explantation des Rattenherzens wurden mittels standardisierter Verfahren RNA isoliert und aufgereinigt. 2µg RNA und Random-Primer (Roche Diagnostics) werden für 10min bei 70°C erhitzt, um anschließend die reverse Transkription zu initiieren (60min bei 42°C). Es werden zu Random-Primern und RNA Pufferlösung (Promega), dNTPs (1nM, Roche Diagnostics), Rnase Inhibitor (40U, Stratagene) und AMV-Reverse Transkriptase (20U, Promega) hinzupipettiert. Die Reaktion wird durch Erhitzen auf 95°C für 5min gestoppt.
Im Anschluss wird in eine LightCycler Kapillare das erfindungsgemäße PCR-Gemisch hinzugegeben. Dazu werden je 400nM der spezifischen Oligonukleotid Primer und die entsprechende copyDNA hinzupipettiert (10ng und 1ng).
Die PCR-Reaktion wird im Anschluss an eine 2 minütige Denaturierungsphase bei 95°C gestartet. Danach werden 55 Zyklen mit folgendem Ablauf wiederholt: 95°C für 0s, dann 57°C für 8s und 72°C für 12s. Bei 86°C erfolgte bei jedem Zyklus pro Kapillare eine aktuelle Fluoreszenzmessung, aus der sich die in Abbildung 18 dargestellte Echt-Zeit-PCR Kurve ergibt. Im Anschluss an die PCR-Reaktion wird eine Schmelzpunktanalyse durchgeführt. Dazu werden die DNA-Syntheseprodukte auf 64°C erwärmt und langsam (0,2°C/s) auf 98°C erhitzt. Während dieser Zeitspanne wird online die Fluoreszenz ermittelt.
Für das jeweilige DNA-Syntheseprodukt wird eine spezifische Schmelztemperatur erreicht, wodurch sich die angelagerten fluoreszierenden SYBR®-Green Moleküle von den schmelzenden DNA-Strängen ablösen, womit sich die Fluoreszenzausbeute schlagartig verringert. Das Fluoreszenzverhalten wird durch eine spezielle Software in Schmelzkurven umgerechnet, welche dann als erste negative Ableitung in Abhängigkeit von der Temperatur (-dF/dT vs T) graphisch angegeben werden (siehe Abbildung 18).

### Einstellung des optimalen PCR-Reaktionsgemisches

### a) Einstellung der optimalen SYBR®-Green-Konzentration

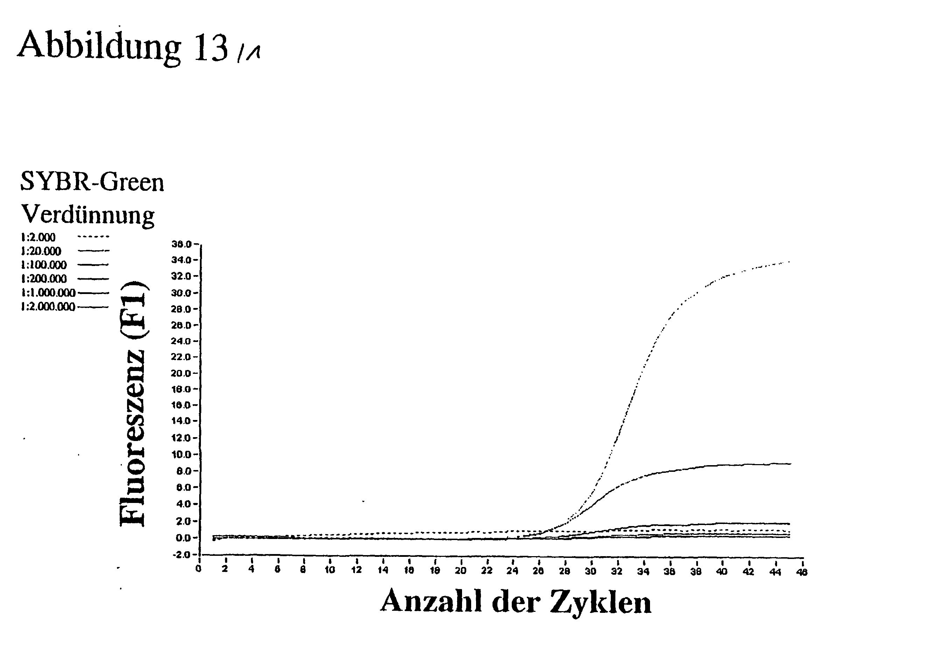

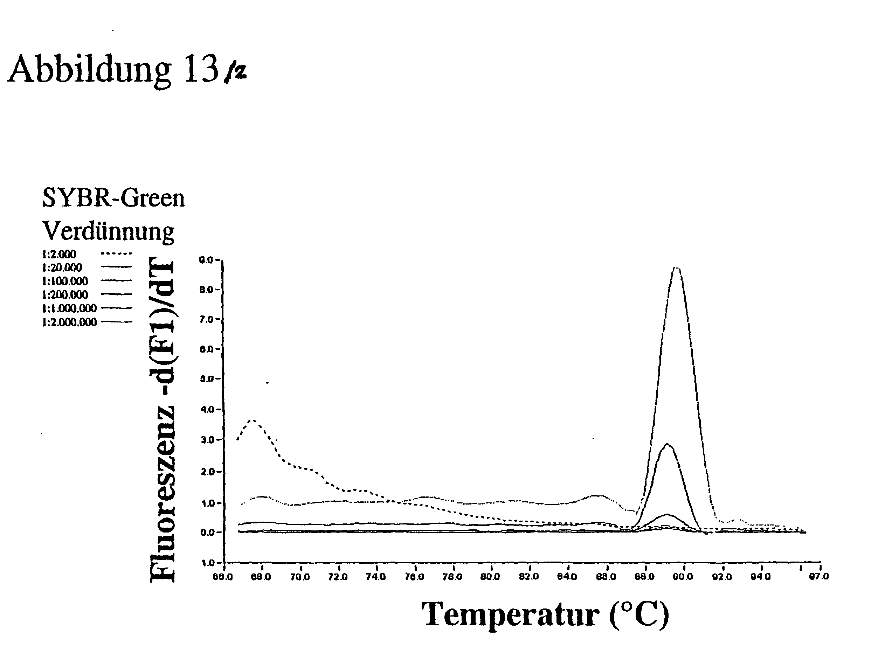

Getestet wurden SYBR®-Green Verdünnungen von 1:2.000 bis 1:2.000.000 aus der SYBR®-Green Stammlösung. Es wurde die sarcoplasmatischen Calcium-ATPase aus gewonnener cDNA des humanen Herzens amplifiziert. Die genauen Versuchsbedingungen sind unter Beispiel 2 beschrieben. Der größte meßbare Fluoreszenzanstieg und damit das beste Ergebnis wurde bei einer Verdünnung von 1:20.000 erzielt. Dadurch wird die Dynamik während der Amplifikation und beim Abschmelzen bei einer Verdünnung von 1:20.000 am größten, so dass auch bei der Schmelzpunktanalyse die Amplitude der durch den Einsatz einer SYBR®-Green-Verdünnung von 1:20.000 erzeugten Schmelzkurve am höchsten ist, wodurch eineindeutige Befundungen erzielt werden. Andere Verdünnungsstufen, die über oder unter der Verdünnung von 1:20.000 lagen, ergaben deutlich schlechtere Ergebnisse, bishin zur fehlenden Detektion (siehe Abbildung 13).

### b) Einstellung der optimalen MgCl₂-Konzentration

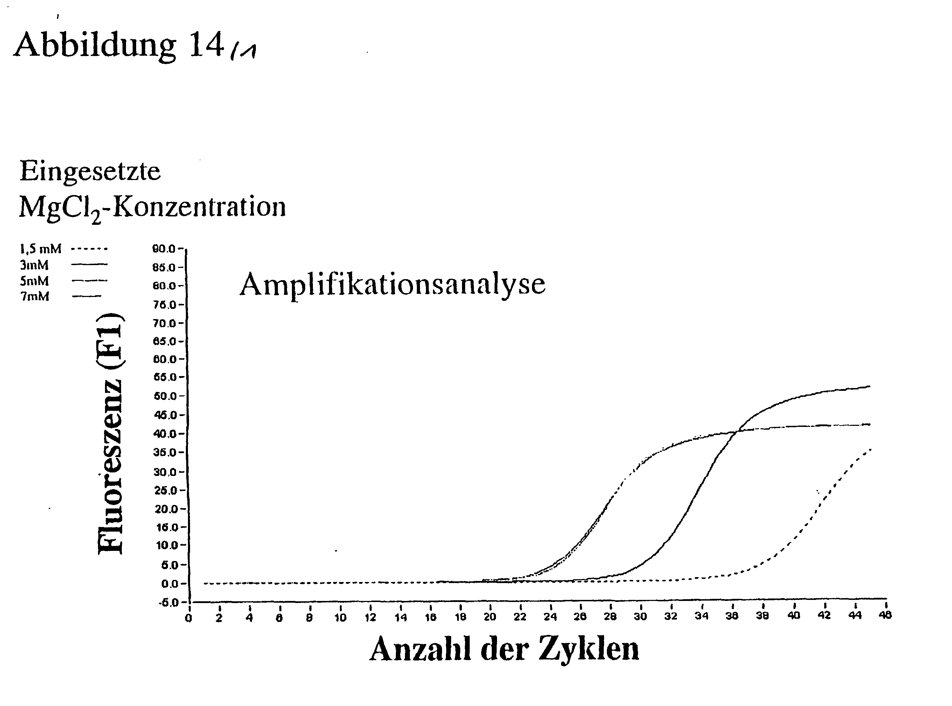

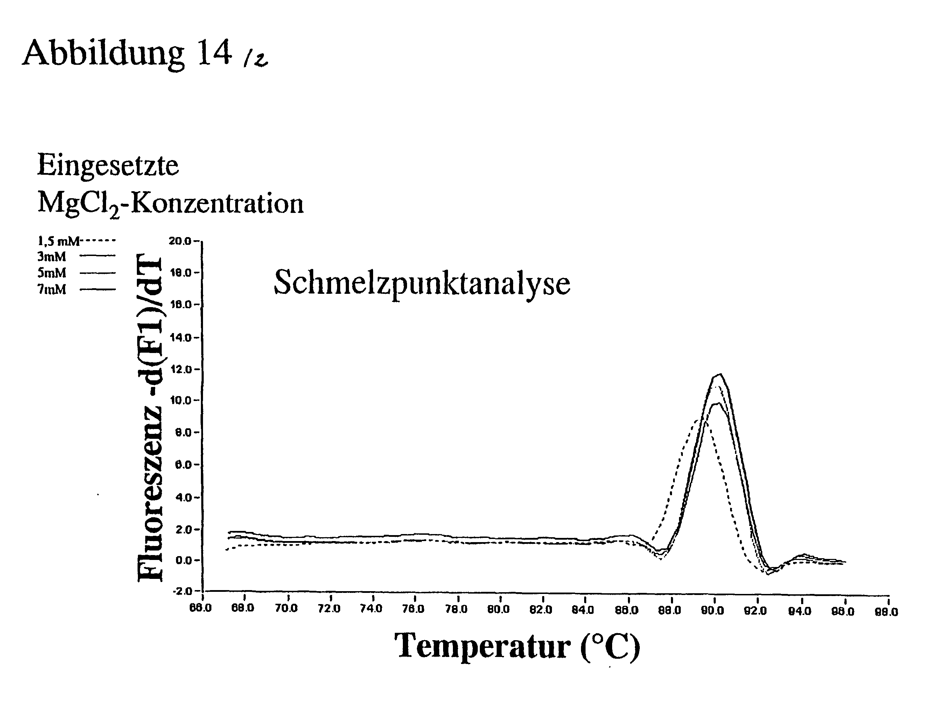

Eine Erhöhung der MgCl₂-Konzentration von 1,5mM über 3mM auf 5mM führte zu einer konsequenten Verkürzung des Beginns der exponentiellen (log) DNA-Synthese, wie anhand der geringeren PCR-Zykluszahlen dokumentiert wird (siehe Abbildung 13). Eine zusätzliche Erhöhung der MgCl₂-Konzentration auf 7mM führte zu keiner Verbesserung in der Analytik. Fluoreszenzanstiegs.
Die Amplituden (Höhe) der entstandenen Schmelzkurven sind bei einer MgCl2 Konzentation von 3, 5 und 7mM vergleichbar. Die beobachtete Verschiebung der Schmelzkurven zu höheren Temperaturen ist auf die veränderte MgCl₂-Konzentation zurückzuführen (siehe Abbildung 14).

### c) Einstellung der optimalen BSA-Konzentration

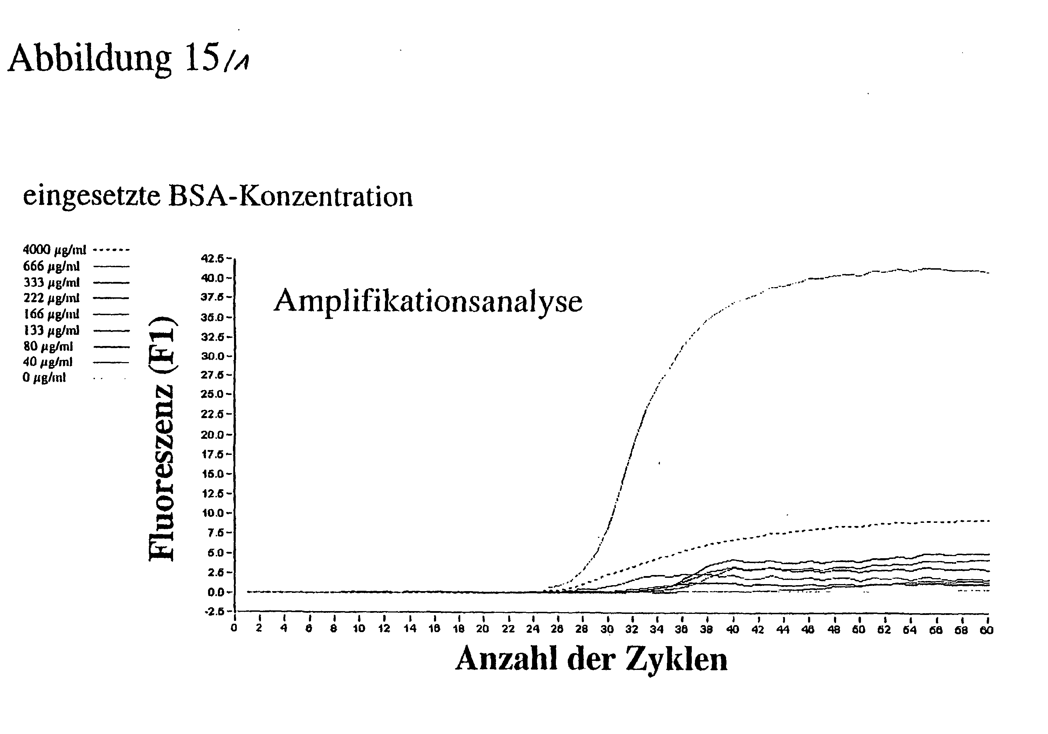

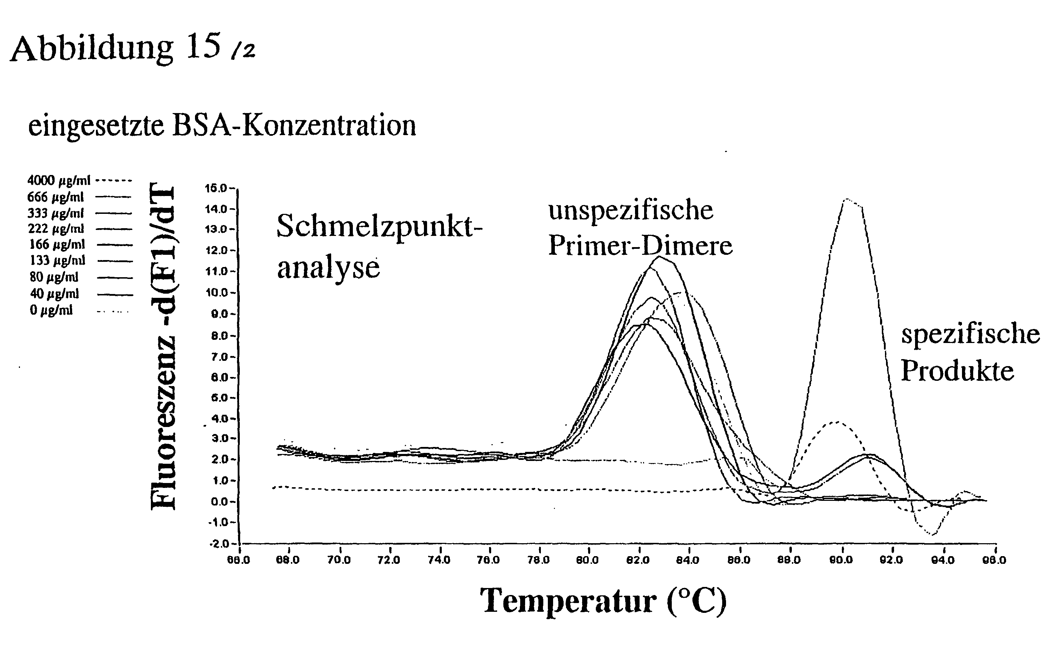

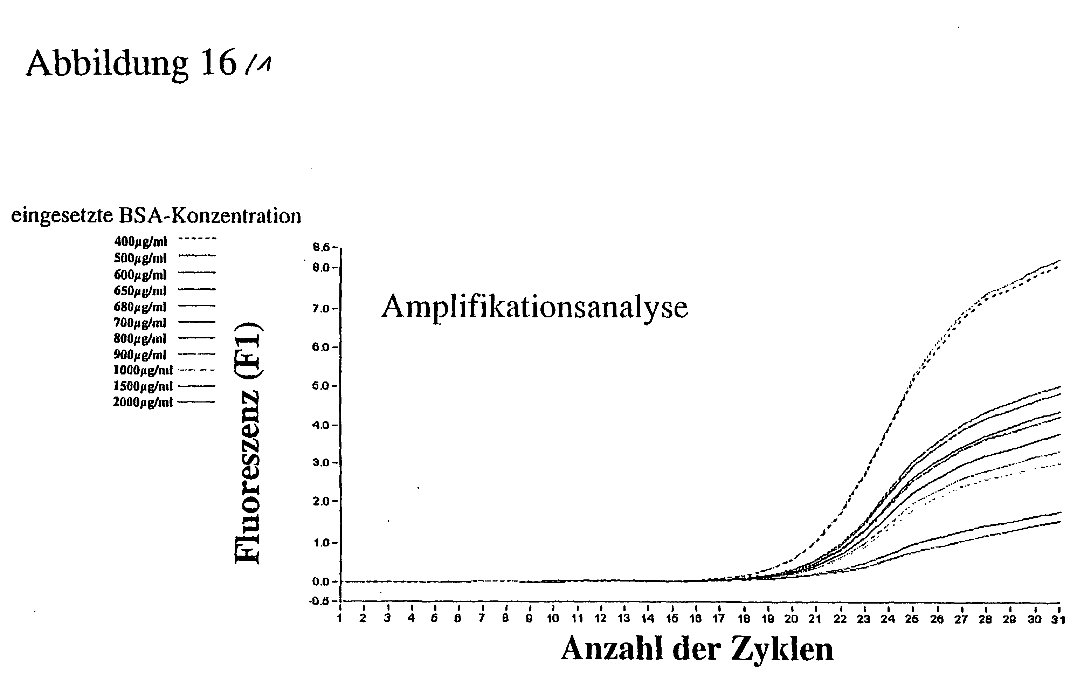

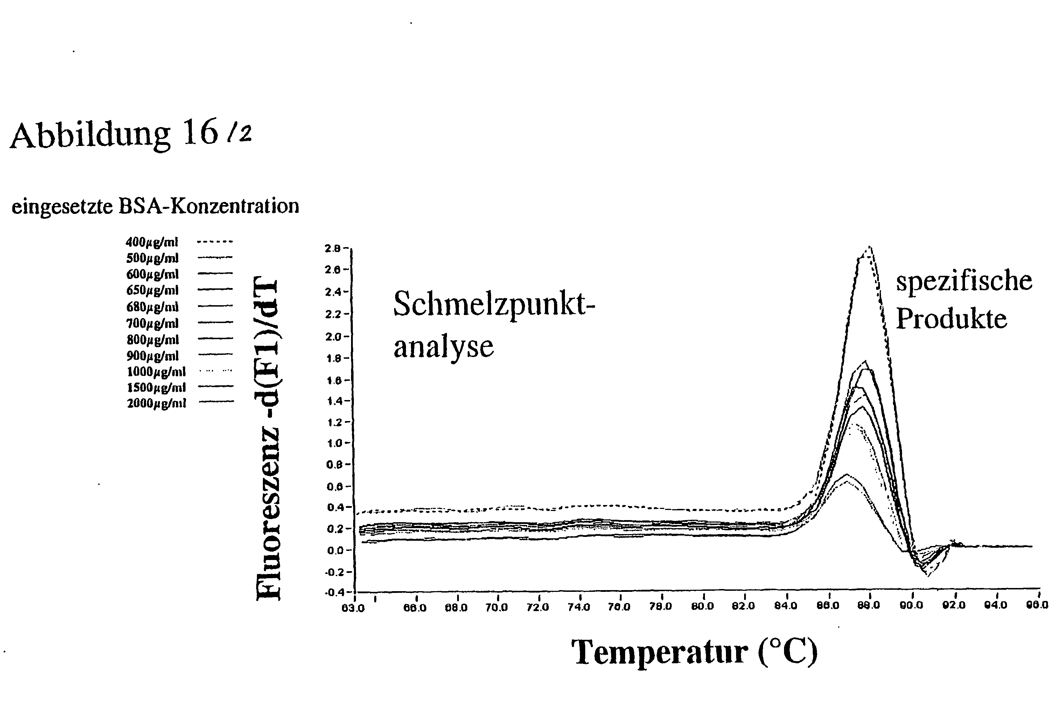

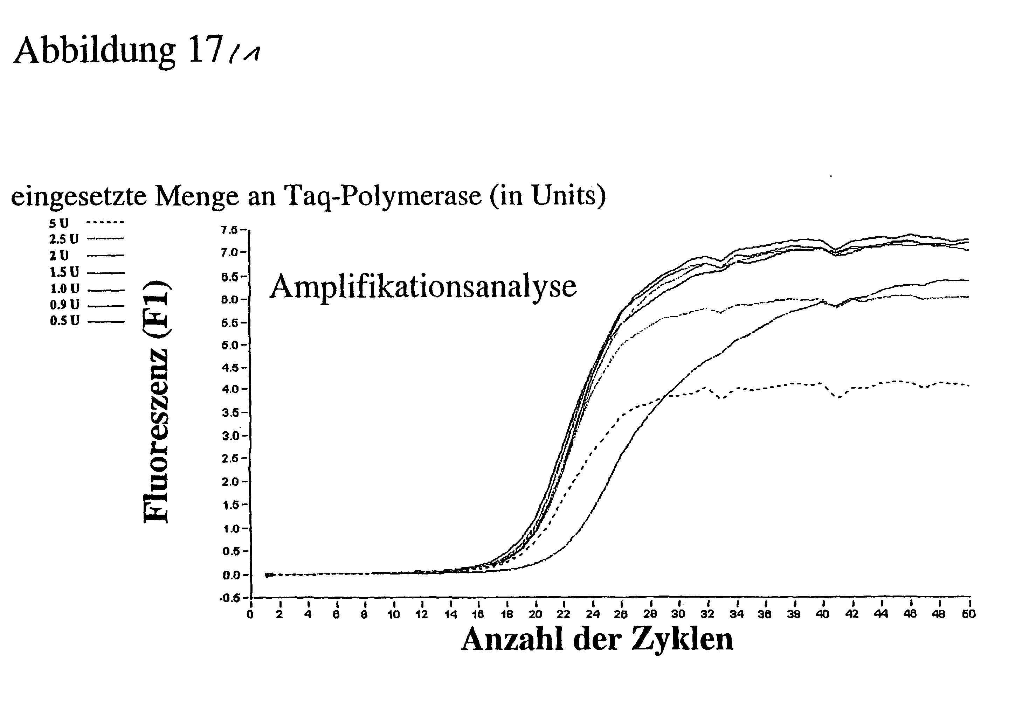

Das beste Ergebnis ließ sich bei einer Konzentration von ca. 500µg/ml BSA erzielen (siehe Abb. 16). Bei dieser Konzentration ist die Dynamik des Fluoreszenzanstiegs und des Abschmelzens am größten. Im Gegensatz zu den Roche Diagnostics und QuantiTect Qiagen Kits entstehen im erfindungsgemäßen Verfahren bei einer BSA-Konzentration von 500µg/ml in der PCR-Reaktion keine unspezifischen Primer-Dimere, die zu einer Fehlbeurteilung führen können. Wenn die BSA-Konzentration nicht exakt eingestellt ist, kommt es zu ausgeprägten Primer-Dimer-Bildungen (siehe Abbildung 15).

### d) Abhängigkeit der verwendeten Taq-Polymerase

Es wurden Taq DNA Polymerasen von verschiedenen Herstellern (TaqDNA-Polymerase von Roche Diagnostics, Taq DNA Polymerase von Life Technologies, PCR Supermix von Life Technologies, Platinum Taq Polymerase von Life Technologies, Taq DNA Polymerase von Promega, Taq DNA Polymerase von TaKaRa, Taq DNA Polymerase von Qiagen, HotStart Taq DNA Polymerase Mastermix von Qiagen) untersucht. Mit Taq-Polymerasen anderer Anbieter ist unser beanspruchtes Hersteller auch durchführbar.

### e) Abhängigkeit von der eingesetzten Unitmenge an Taq-Polymerase

Getestet wurde die Unitmenge 5U, 2.5U, 2.0U, 1.5U, 1.0U, 0.9U und 0.5U. Wie aus Abbildung 17 ersichtlich ließen sich mit unserem beanspruchten Verfahren mit den Unitmengen 0.9U bis 2.5 U gleich gute Ergebnisse erzielen. Auch mit 0.5U TaqPolymerase / Reaktion konnte die Reaktion ohne Einbußen in der Spezifität durchgeführt werden, lediglich der dynamische Amplifikationsanstieg war zu etwas höheren Zykluszahlen verschoben (siehe Abb. 17).

Der Tabelle 1) ist eine vergleichende Darstellung der Zusammensetzung des erfindungsgemäßen PCR-Reaktionsgemisches unter Verwendung des HotStarTaq Master Mix von Qiagen GmbH (HotStart Variante) und der Standard-PCR Variante (s.o.) mit den kommerziell angebotenen Kits der Roche Diagnostics GmbH und Qiagen (QuantiTect) zu entnehmen.

### In Tabelle 2) sind die Vorteile der Erfindung und die Nachteile der Kits des Standes der Technik (Roche Diagnostics GmbH) dargestellt.

### Anmerkungen zum QuantiTect SYBR®-Green PCR Kit der Firma Qiagen

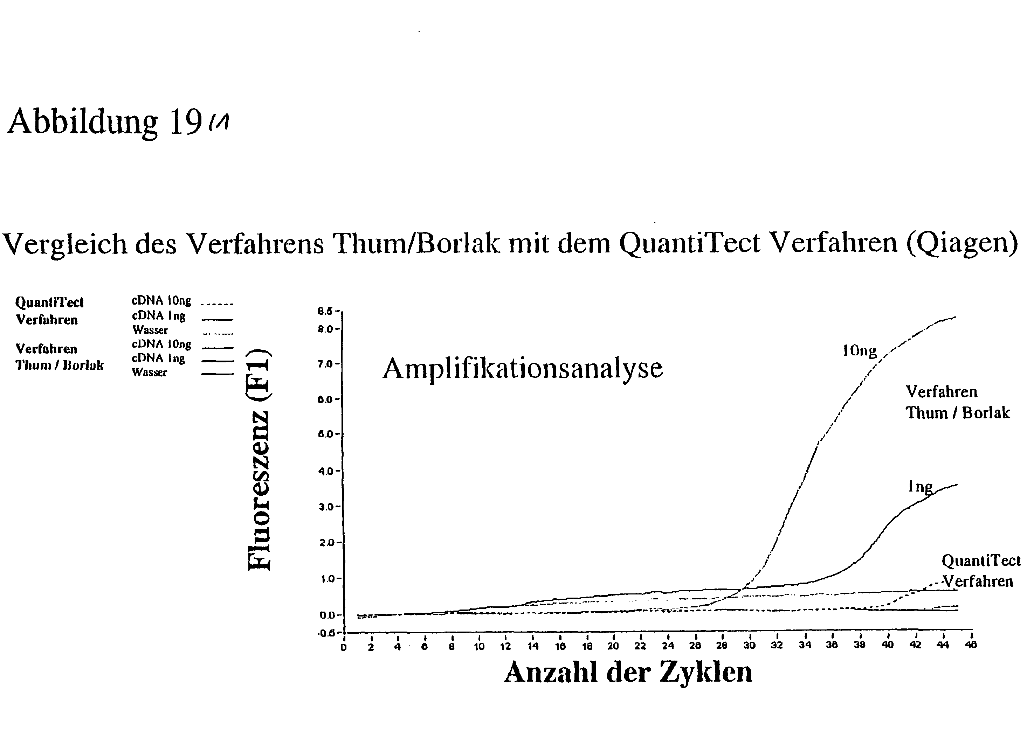

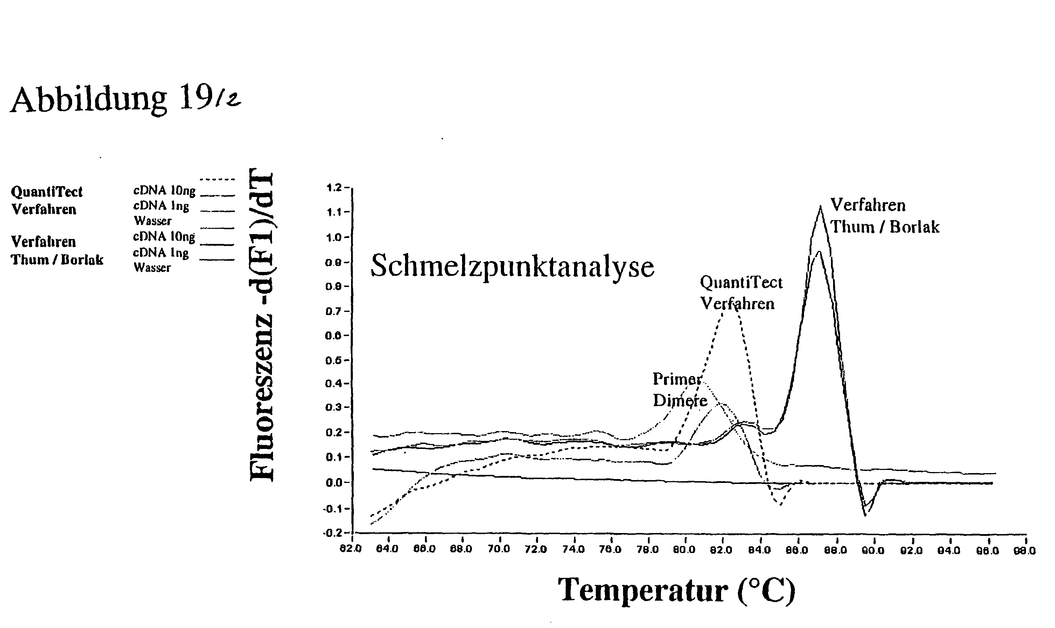

Im Vergleich zum Verfahren mit einem erfindungsgemäßen Gemisch (HotStart und normale PCR Variante) wurden deutlich schlechtere Ergebnisse erzielt (siehe Abb.19). Bei der Amplifikation des CYP 1A1 Gens aus cDNA kultivierter Hepatozyten war die Fluoreszenzausbeute des QuantiTect Verfahrens etwa 10 fach geringer im Vergleich zum erfindungsgemäßen Verfahren. Während erfindungsgemäß keine unspezifischen Primer-Dimere entstehen, konnte bei der Nutzung des QuantiTect Verfahrens die quantitativ bedeutsame Bildung unspezifischer Dimere beobachtet werden, wodurch Fehlbeurteilungen möglich sind, zumindest aber die quantitative Bewertung der Genexpression beeinträchtigt wird und damit definitiv falsche Ergebnisse erzielt werden.

Die Schmelzhügel der spezifischen Produkte waren im erfindungsgemäßen Verfahren deutlich höher, so dass bei höheren Temperaturen (höhere Spezifität) höhere Fluoreszenzausbeuten bessere und exaktere Befundungen im Vergleich zu dem QuantiTect Verfahren der Firma Qiagen gezeigt werden können (siehe auch Abb. 19). Eine genaue Befundung unter Benutzung des QuantiTect Systems ist so gut wie unmöglich, da Schmelzhügel der spezifischen Produkte und Primer Dimere sehr eng zusammen liegen (innerhalb von 1-2°C), wodurch erhebliche Risiken in der Beurteilung der Genamplifikationsprodukte entstehen. Nach Rücksprache mit dem technischen Service der Firma Qiagen wurde berichtet, daß die spezifischen Produkte im Vergleich zu dem Roche Kit ca. 3 - 8°C niedriger liegen. Da potentiell entstehende Primer Dimere gewöhnlich bei Temperaturen von 70 - 80°C abschmelzen, ist eine exakte Identifizierung bei den meisten der amplifizierten Gene aufgrund des zu niedrigeren Temperaturen verschobenen Schmelzhügels unter Nutzung des QuantiTect Systems nicht möglich. Ein typisches Beispiel liefert Abbildung 19. Eine Gen-Amplifizierung ist laut Produktinformation des QuantiTect Verfahrens der Firma Qiagen nur möglich, wenn die Denaturierungsphase innerhalb eines PCR-Zyklusses mindestens 15s beträgt und die Annealingphase mindestens 20s. Dadurch kann der zeitliche Ablauf im Vergleich zu dem beanspruchten Verfahren um bis zu 100% verlängert werden (siehe auch QuantiTect SYBR®-Green PCR Handbook, Stand Juni 2001, Firma Qiagen).

### Beschreibungen der Abbildungen

Abbildung 1) Darstellung des Fluoreszenz-Resonanz-Energie-Transfer (FRET)-Prinzips. Fluoreszenz-gelabelte Hybridisierungssonden binden an die komplementäre DNA. Im Falle eines Nukleotidpolymorphismus (siehe Markierung) hat die entsprechende Hybridisierungssonde ein anderes Schmelzverhalten und der Nukleotidpolymorphismus wird somit detektiert.
Abbildung 2) Darstellung des SBYR-Green I Labelings. Der fluoreszierende Farbstoff SYBR®-Green I interkaliert zwischen doppelstängige DNA und die PCR-synthetisierte DNA-Menge korreliert mit dem zunehmenden Fluoreszenzsignal.
Abbildung 3) Schmelzkurvenanalyse nach Amplifikation des Transkriptionsfaktors HNF3 α aus cDNA kultivierter Hepatozyten der Ratte. Die Abbildung dokumentiert die Bildung von unspezifischen Primer-Dimeren nach Verwendung des Roche Diagnostics-LightCycler-FastStart-DNA-Master-Hybridisation Probes-Kits. In der erfindungsgemäßen Verwendung kann gezeigt werden, dass unter sonst gleichen Bedingungen die Bildung von unspezifischen Primer-Dimeren unterdrückt wird. Die Bildung von unspezifischen Primer-Dimeren kann zu Fehldiagnosen und Fehlinterpretationen führen.
Abbildung 4) Nachweis einer Genpolymorphie in Morbus Meulengracht Patienten. Mittels FRET-Technologie wurde der Wildtyp des UGT1A1 Gens diagnostiziert. Mit dem beanspruchten Verfahren kann aus 1pg eingesetzter lymphozytärer DNA die Genpolymorphie, die dem Morbus Meulengracht zugrunde liegen eindeutig identifiziert werden, so dass das erfindungsgemäße Gemisch im Vergleich zu den Roche Diagnostic Kits eine 10.000fach höhere Sensitivität aufweist.
Abbildung 5) Nachweis der sarcoplasmatischen Calcium-ATPase aus gewonnener cDNA von humanen Herzen unter Einsatz des beanspruchten Verfahrens.. Die Abbildung zeigt die Amplifikation und die Schmelzkurve dieses Gens. Mit dem erfindungsgemäßen Gemisch werden wesentlich höhere Fluoreszenzausbeuten erzielt, die eine verbesserte und quantitative Befundung des krankheitsrelevanten Genes ermöglicht.
Abbildung 6) Nachweis des beta-myosin-heavy-chain- (MHC) Gens aus gewonnener cDNA von humanem Herzgewebe unter Einsatz des erfindungsgemäßen Gemisches. Die Abbildung zeigt die Amplifikation und die Schmelzkurvenanalyse dieses Gens bei Einsatz von 3 verschiedenen Ausgangsverdünnungen der cDNA.
Abbildung 7) Nachweis des brain natriuretic peptide (BNP) Gens aus gewonnener cDNA von humanem Herzgewebe. Die Abbildung zeigt die Amplifikation und die Schmelzkurvenanalyse dieses Gens bei Einsatz von 3 verschiedenen Ausgangsverdünnungen der cDNA..
Abbildung 8) Nachweis des atrialen natriuretischen Peptid (ANP) Gens aus cDNA von kultivierten Kardiomyozyten der Ratte unter Einsatz des beanspruchten Verfahrens. Die Abbildung zeigt die Amplifikation und die Schmetzkurvenanalyse dieses Gens bei Einsatz von 3 verschiedenen Ausgangsverdünnungen der cDNA.
Abbildung 9) Nachweis des alpha skelettalen Aktin Gens aus cDNA von kultivierten Kardiomyozyten der Ratte unter Einsatz des erfindungsgemäßen Gemisches. Die Abbildung zeigt die Amplifikation und die Schmelzkurvenanalyse dieses Gens bei Einsatz von 3 verschiedenen Ausgangsverdünnungen der cDNA.
Abbildung 10) Nachweis des Albumin Gens aus cDNA von kultivierten Hepatozyten der Ratte unter Einsatz des beanspruchten Verfahrens. Die Abbildung zeigt die Amplifikation und die Schmelzkurvenanalyse dieses Gens bei Einsatz von 3 verschiedenen Ausgangsverdünnungen der cDNA.
Abbildung 11) Nachweis des Transkriptionsfaktors HNF-3gamma aus cDNA von kultivierten Hepatozyten der Ratte unter Einsatz des erfindungsgemäßen Gemisches. Die Abbildung zeigt die Amplifikation und die Schmelzkurvenanalyse dieses Gens bei Einsatz von 3 verschiedenen Ausgangsverdünnungen der cDNA.
Abbildung 12) Nachweis des N-Acetyltransferase 2 Allel 5* Genotyps aus humaner lymphozytärer DNA mit FRET-Hybridisierungssonden unter Einsatz des beanspruchten Verfahrens. Das beanspruchte Verfahren ermöglicht die exakte Zuordung von Wildtyp und mutierten Genotypen. Im Falle einer Gen-Mutation schmilzt die Hybridisierungssonde 8°C niedriger ab, womit eine exakte Zuordung der Genotypen ermöglicht wird.
Abbildung 13)Darstellung der Konzentrationsabhängigkeit des eingesetzten SYBR®-Green I Labelings im beanspruchten Verfahren. Bei einer Konzentration von 1:20000 wurde das beste Ergebnis erzielt.
Abbildung 14) Darstellung der Konzentrationsabhängigkeit des eingesetzten MgCl₂ im beanspruchten Verfahren. Bei einer Konzentration von 5mM wurde das beste Ergebnis erzielt.
Abbildung 15) Darstellung der Konzentrationsabhängigkeit des eingesetzten BSA (bovines Serum Albumin) im erfindungsgemäßen Gemisch.
Abbildung 16) Darstellung der Konzentrationsabhängigkeit des eingesetzten BSA (bovines Serum Albumin) im erfindungsgemäßen Gemisch. Es wurden die Konzentrationen 400µg/ml bis 2000µg/ml in engen Abständen untersucht. Bei einer Konzentration von 500µg/ml wurde das beste Ergebnis erzielt.
Abbildung 17) Darstellung der Abhängigkeit der verwendeten Unitmenge an Taq-DNA Polymerase. Getestet wurden die Mengen 0.5U bis 5U. Bei einer Konzentration von 0.9U Taq-Polymerase konnten die besten Ergebnisse erzielt werden.
Abbildung 18) Nachweis des CYP2J3 Gens aus cDNA explantierter Rattenherzen unter Einsatz des beanspruchten Verfahrens. Die Abbildung zeigt die Amplifikation und die Schmelzkurvenanalyse dieses Gens unter Verwendung von 2 verschiedenen Ausgangsverdünnungen der cDNA.
Abbildung 19) Vergleich des Verfahrens mit einem erfindungsgemäßen Gemisch mit dem QuantiTect Verfahren der Firma Qiagen. Wie aus Abbildung 19 ersichtlich, ist die Sensitivität und Genauigkeit der Erfindung deutlich überlegen. Eine Identifizierung von Genamplifikaten unter Nutzung des QuantiTect Verfahrens der Firma Qiagen ist aufgrund ähnlicher Schmelzhügel von spezifischen Produkt und Primer Dimeren nicht möglich. Unter Verwendung des erfindungsgemäßen Gemisches ist eine Identifizierung ohne Probleme durchführbar.

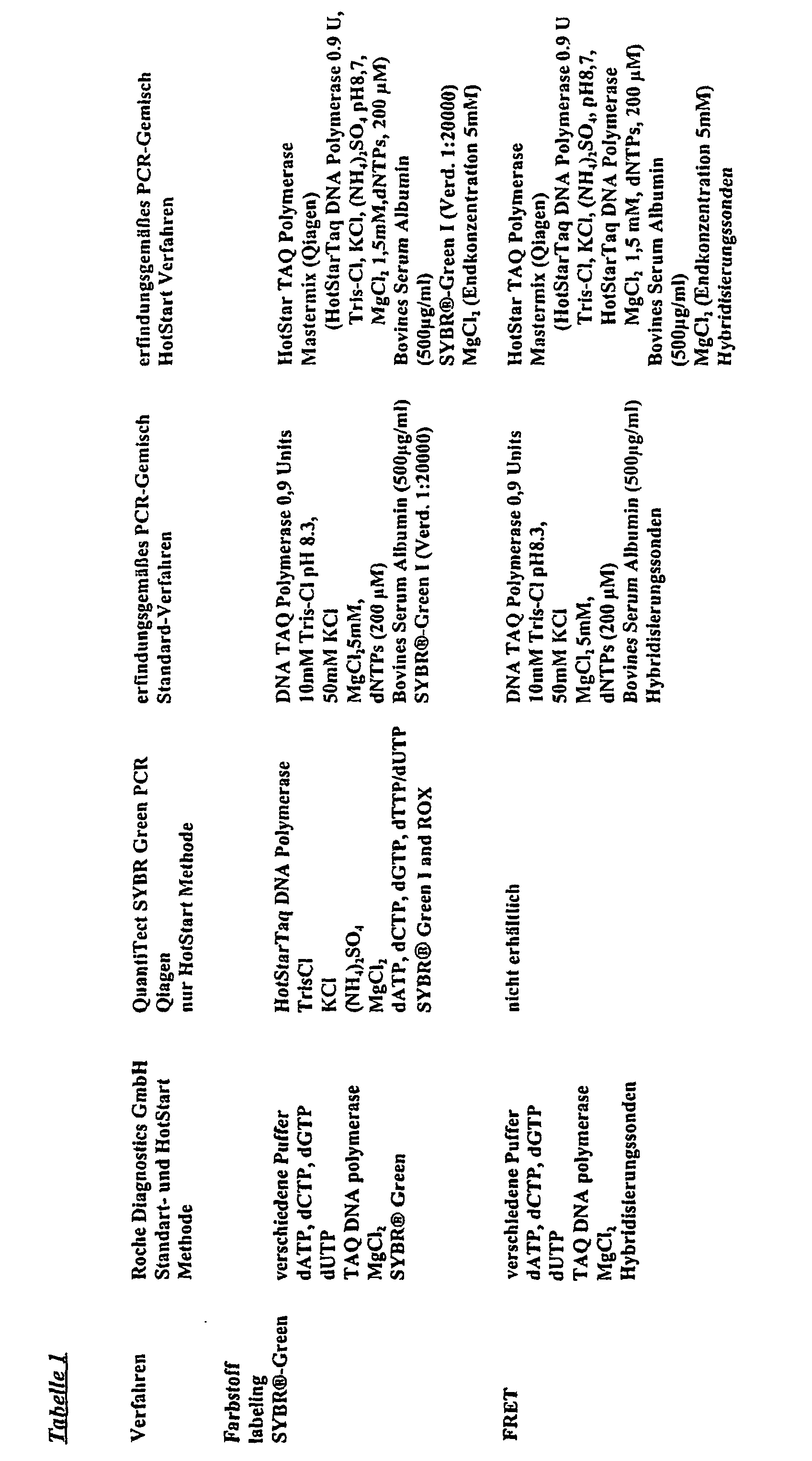

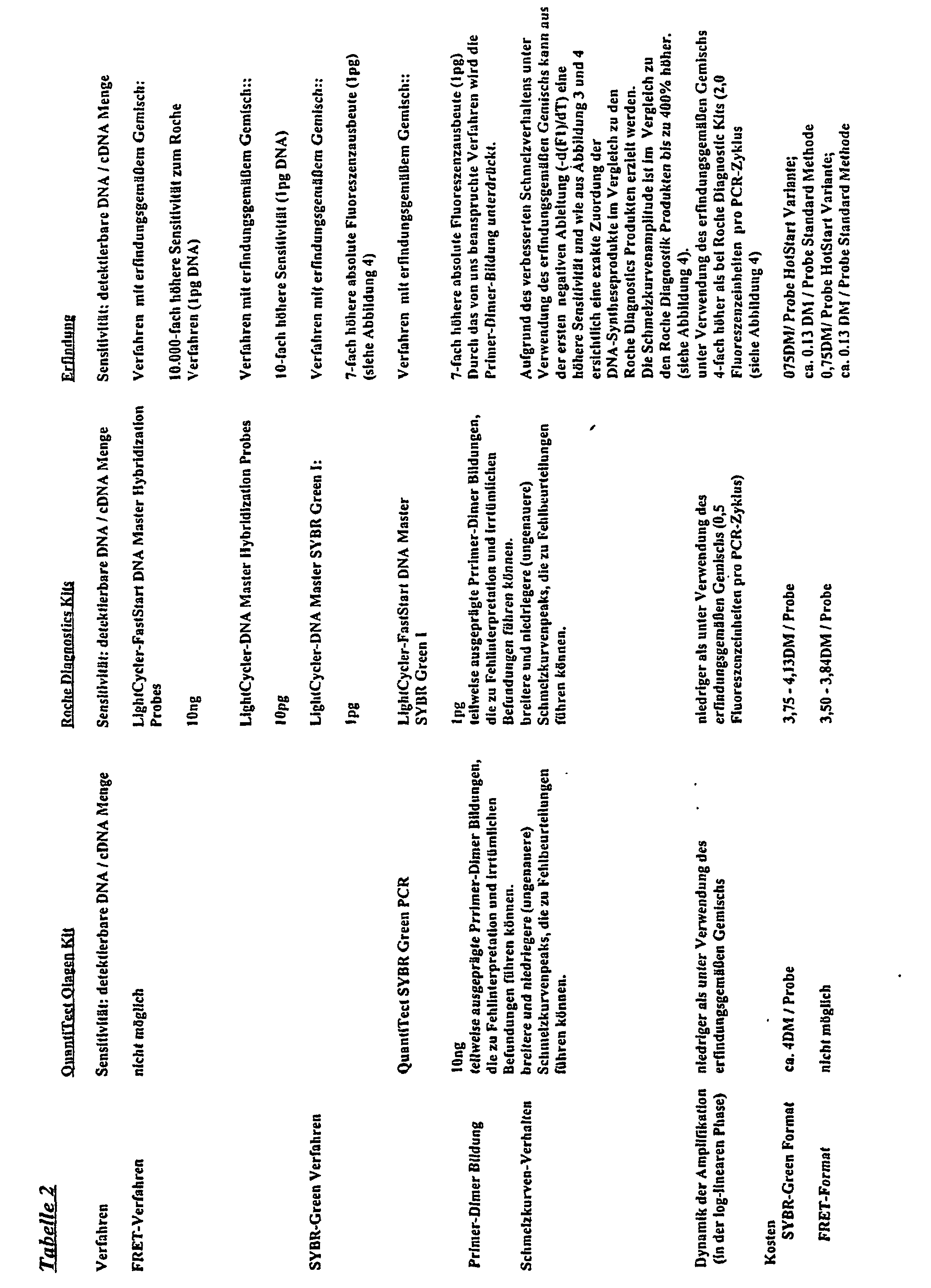

## Patentansprüche

1. PCR-Reaktionsgemisch für Fluoreszenz-basierende Genexpressions- und Genmutationsanalysen umfassend
- eine beliebige Taq-Polymerase,
- BSA (Bovines Serum Albumin), welches im Reaktionsgemisch in einer Endkonzentration im Bereich von 400 bis 800 µg/ml vorliegt,
- MgCl₂ in einer Endkonzentration von > 3,5 mM bis einschließlich 5 mM sowie
- ein fluoreszierendes Material.

2. PCR-Reaktionsgemisch nach Anspruch 1 umfassend
- BSA in einer Endkonzentration von 500 µg/ml, und
- MgCl₂ in einer Endkonzentration von 5 mM.

3. PCR-Reaktionsgemisch nach Anspruch 1 oder 2 **gekennzeichnet durch** die folgenden Reaktionskomponenten:
• Tris-Cl, KCl, dNTPs, (NH₄)₂SO₄,
• DNA Taq-Polymerase,
• MgCl_{2,}
• BSA,
• einen fluoreszierenden Farbstoff oder Fluoreszenzsonden, mit denen Fluoreszenz-Resonanz-Energie-Transfer (FRET) basierte Analysen möglich sind.

4. Verwendung eines PCR-Reaktionsgemisches gemäß einem der Ansprüche 1 bis 3 zur Fluoreszenz-basierenden Genexpressions- und Genmutationsanalyse in vitro, wobei DNA oder RNA aus biologischen Materialien isoliert wird, die RNA in cDNA umgewandelt wird, eine PCR-Amplifikation mit spezifischen Oligonukleotidprimern erfolgt und anschließend in Abhängigkeit des Farbstoff-Labelings oder der Sonden die Auswertung erfolgt.

5. Verwendung nach Anspruch 4 zum Nachweis von Nukleotid-Polymorphismen; für die Charakterisierung von potentiellen Arzneistoffen auf der Genexpressionsebene; zur Pharmakogenomic und Toxikogenomic; zur molekularen Diagnostik; zur Erkennung von molekularen Interaktionen von Stoffen, Stoffgemischen und biologischen Agenzien auf der Genomebene, zur Erkennung von Gen-Interaktionen und Funktionsanalyse neuer Gene, einschließlich Sequenzanalysen und Genklonierungen.

6. Verwendung nach Anspruch 4 oder 5 zum Nachweis einer Genpolymorphie in Morbus Meulengracht Patienten.

7. Verwendung nach Anspruch 4 oder 5 zum Nachweis der sarcoplasmatischen Calcium-ATPase aus gewonnener cDNA von humanen Herzen.

8. Verwendung nach Anspruch 4 oder 5 zum Nachweis des beta-myosin-heavy-chain-(MHC) Gens aus gewonnener cDNA von humanem Herzgewebe.

9. Verwendung nach Anspruch 4 oder 5 zum Nachweis des brain natriuretic peptide (BNP) Gens aus gewonnener cDNA von humanem Herzgewebe.

10. Verwendung nach Anspruch 4 oder 5 zum Nachweis des atrialen natriuretischen Peptid (ANP) Gens aus cDNA von kultivierten Kardiomyozyten.

11. Verwendung nach Anspruch 4 oder 5 zum Nachweis des alpha skelettalen Aktin Gens aus cDNA von kultivierten Kardiomyozyten.

12. Verwendung nach Anspruch 4 oder 5 zum Nachweis des Albumin Gens aus cDNA von kultivierten Hepatozyten.

13. Verwendung nach Anspruch 4 oder 5 zum Nachweis des Transkriptionsfaktors HNF-3gamma aus cDNA von kultivierten Hepatozyten.

14. Verwendung nach Anspruch 4 oder 5 zum Nachweis des N-Acetyltransferase 2 Allel 5* Genotyps aus humaner lymphozytärer DNA.

15. Verwendung nach Anspruch 4 oder 5 zum Nachweis des CYP2J3 Gens aus cDNA explantierter Rattenherzen.

## Claims

1. PCR reaction mixture for fluorescence-based gene expression and gene mutation analyses entailing
- an arbitrary Taq polymerase,
- BSA (Bovine Serum Albumin), existent in the reaction mixture in a final concentration in the range from 400 to 800 µg/ml,
- MgCl₂ in a final concentration of > 3.5 mM up to and including 5 mM as well as
- a fluorescent material.

2. PCR reaction mixture in accordance with Claim 1 entailing
- BSA in a final concentration of 500 µg/ml, and
- MgCl₂ in a final concentration of 5 mM.

3. PCR reaction mixture in accordance with Claim 1 or 2 wherein there exist the following reaction components:
• Tris-Cl, KCl, dNTPs, (NH₄)₂SO_{4,}
• DNA Taq polymerase,
• MgCl_{2,}
• BSA,
• a fluorescent dye or fluorescence probes, with which fluorescence resonance energy transfer (FRET) based analyses are possible.

4. Use of a PCR reaction mixture in accordance with one of the claims 1 to 3 for fluorescence-based gene expression and gene mutation analysis in vitro, with DNA or RNA being isolated from biological materials, the RNA being converted into cDNA, a PCR amplification taking place with specific oligonucleotide primers and subsequently the evaluation taking place as a function of the dye labelling or the probes.

5. Use in accordance with Claim 4 to detect nucleotide polymorphisms; for the characterisation of potential drugs on the gene expression level; for pharmacogenomics and toxicogenomics; for molecular diagnostics; for recognition of molecular interactions of materials, mixtures of materials and biological agents on the genome level, to recognise gene interactions and function analysis of new genes, including sequence analyses and gene cloning.

6. Use in accordance with Claim 4 or 5 to detect a gene polymorphia in Morbus Meulengracht patients.

7. Use in accordance with Claim 4 or 5 to detect the sarcoplasmatic calcium-ATPase from cDNA obtained from human hearts.

8. Use in accordance with Claim 4 or 5 to detect the beta-myosin heavy chain (MHC) gene from cDNA obtained from human heart tissue.

9. Use in accordance with Claim 4 or 5 to detect the brain natriuretic peptide (BNP) gene from cDNA obtained from human heart tissue.

10. Use in accordance with Claim 4 or 5 to detect the atrial natriuretic peptide (ANP) gene from cDNA from cultivated cardiomyocytes.

11. Use in accordance with Claim 4 or 5 to detect the alpha skeletal actin gene from cDNA from cultivated cardiomyocytes.

12. Use in accordance with Claim 4 or 5 to detect the albumin gene from cDNA from cultivated hepatocytes.

13. Use in accordance with Claim 4 or 5 to detect the transcription factor HNF-3gamma from cDNA from cultivated hepatocytes.

14. Use in accordance with Claim 4 or 5 to detect the N-acetyl transferase 2 Allel 5* genotype from human lymphocytary DNA.

15. Use in accordance with Claim 4 or 5 to detect the CYP2J3 gene from cDNA of explanted rats' hearts.

## Revendications

1. Mélange de réaction PCR pour analyses d'expression et de mutation de gène, basées sur la fluorescence, englobant
- un Taq Polymérase quelconque,
- le BSA (sérum bovin à l'albumine) qui se trouve dans le mélange de réaction dans une concentration finale entre 400 et 800 µg/ml,
- MgCl₂ dans une concentration finale de > 3,5 mM à 5 mM inclus ainsi que
- un matériel fluorescent.

2. Mélange de réaction PCR selon la revendication 1 comprenant
- BSA dans une concentration finale de 500 µg/ml et
- MgCl₂ dans une concentration finale de 5 mM.

3. Le mélange de réaction PCR selon la revendication 1 et 2 se **caractérise par** les composantes de réaction suivantes :
• Tris-Cl, KCI, dNTPs, (NH₄)₂SO₄,
• ADN Taq Polymérase,
• MgCl₂,
• BSA,
• un colorant fluorescent ou des sondes de fluorescence avec lesquels les analyses basées sur le transfert d'énergie / résonance par fluorescence (FRET) sont possibles.

4. Emploi d'un mélange de réaction PCR selon l'une des revendications 1 à 3 pour l'analyse d'expression et de mutation de gène, basées sur la fluorescence in vitro, l'ADN ou l'ARN ayant été isolé à partir de matériaux biologiques, l'ARN ayant été transformé en ADNc, une amplification PCR ayant eu lieu avec des amorces d'oligonucléotides spécifiques et l'évaluation ayant eu lieu ensuite en fonction du marquage du colorant ou des sondes.

5. Emploi selon la revendication 4 pour mettre en évidence des polymorphismes de nucléotide ; pour la caractérisation de médicaments potentiels au niveau de l'expression de gène ; pour la pharmacogénomique et la toxicogénomique ; pour le diagnostic moléculaire ; pour la détection d'interactions moléculaires de substances, de mélanges de substances et d'agents biologiques au niveau du génome, pour la détection d'interactions génétiques et l'analyse fonctionnelle de nouveaux gènes, y compris les analyses de séquences et le clonage de gène.

6. Emploi selon la revendication 4 ou 5 pour mettre en évidence une polymorphie génétique chez les patients souffrant de la Maladie de Gilbert (cholémie).

7. Emploi selon la revendication 4 ou 5 pour mettre en évidence la calcium-ATPase sarcoplasmatique d'ADNc extrait de coeurs humains.

8. Emploi selon la revendication 4 ou 5 pour mettre en évidence le gène beta-myosin-heavy-chain (MHC) d'ADNc extrait du tissu cardiaque humain.

9. Emploi selon la revendication 4 ou 5 pour mettre en évidence le gène brain natriuretic peptide (BNC) d'ADNc extrait du tissu cardiaque humain.

10. Emploi selon la revendication 4 ou 5 pour mettre en évidence le gène atrial natriuretic peptid (ANP) d'ADNc extrait de cardiomyocytes cultivés.

11. Emploi selon la revendication 4 ou 5 pour mettre en évidence le gène actine alpha squelettique d'ADNc extrait de cardiomyocytes cultivés.

12. Emploi selon la revendication 4 ou 5 pour mettre en évidence le gène albumine d'ADNc extrait d'hépatocytes cultivés.

13. Emploi selon la revendication 4 ou 5 pour mettre en évidence le facteur de transcription HNF-3 gamma d'ADNc extrait d'hépatocytes cultivés.

14. Emploi selon la revendication 4 ou 5 pour mettre en évidence le génotype N-acétyltransférase 2 allèle 5* de l'ADN lymphocytaire humain.

15. Emploi selon la revendication 4 ou 5 pour mettre en évidence le gène CYP2J3 d'ADNc extrait de coeurs de rats explantés.
